# EUROPEAN PATENT SPECIFICATION

(11) **EP 3 463 274 B1**
(45) Date of publication and mention of the grant of the patent: **07.06.2023**
(21) Application number: 17726648.3
(22) Date of filing: 01.06.2017
(51) Int. Cl.: A61K 8/39, A61K 8/46, A61K 8/58, A61Q 5/02, A61Q 5/12, A61K 8/898, A61K 8/90

(54) **COMPOSITION COMPRISING AN ANIONIC SURFACTANT, AN ORGANOSILANE AND POLYOXYALKYLENATED OR QUATERNISED AMINO SILICONE, AND COSMETIC TREATMENT PROCESS**
ZUSAMMENSETZUNG MIT EINEM ANIONISCHEN TENSID, EINEM ORGANOSILAN UND POLYOXYALKYLENIERTEN ODER QUATERNIERTEN AMINOSILIKON UND VERFAHREN ZUR KOSMETISCHEN BEHANDLUNG
COMPOSITION COMPRENANT UN TENSIOACTIF ANIONIQUE, UN ORGANOSILANE ET UN AMINO-SILICONE POLYOXYALKYLÉNÉ OU QUATERNISÉ, ET MÉTHODE DE TRAITEMENT COSMÉTIQUE

(30) Priority: 02.06.2016 FR 1655035
(43) Date of publication of application: 10.04.2019
(73) Proprietor: L'OREAL, 75008 Paris (FR)
(72) Inventor: MINOU, Patrick, 93400 Saint-Ouen (FR); CHESNEAU, Laurent, 93400 Saint-Ouen (FR); ALVES, Diego, 93400 Saint-Ouen (FR)
(74) Representative: L'Oreal
(86) International application number: PCT/EP2017/063288
(87) International publication number: WO 2017/207685

(56) References cited:
- FR-A1- 2 954 099
- US-A- 5 302 322
- US-A1- 2011 158 927
- US-A1- 2012 294 819
- DATABASE GNPD [Online] MINTEL; April 2013 (2013-04), "Shampoo", XP002763762, Database accession no. 2053023
- DATABASE GNPD [Online] MINTEL; November 2015 (2015-11), "Shampoo", XP002763763, Database accession no. 3570147
- DATABASE GNPD [Online] MINTEL; February 2013 (2013-02), "Hydrating shampoo", XP002763764, Database accession no. 2004828
- anonymous: "Silsoft A+ conditioning agent", Momentive , December 2009 (2009-12), XP002763765, Retrieved from the Internet: URL:http://s3.amazonaws.com/zanran_storage /www.momentive.com/ContentPages/45675670.p df [retrieved on 2016-11-03]

## Description

The present invention relates to a composition, in particular a cosmetic composition, in particular a cosmetic hair composition, comprising at least one anionic surfactant, at least one organosilane and at least one particular amino silicone, and also to a cosmetic treatment process for keratin materials using this composition. These compositions are more particularly intended for cleansing and/or conditioning keratin materials, in particular the hair.

It is common practice to use detergent compositions (such as shampoos) based essentially on standard surfactants in particular of anionic, non-ionic and/or amphoteric type, but more particularly of anionic type, for cleansing and/or washing keratin materials such as the hair. These compositions are applied to wet hair and the foam generated by massaging or rubbing with the hands makes it possible, after rinsing with water, to remove the diverse types of soiling initially present on the hair or the skin.

These base compositions do, admittedly, have good washing power, but their intrinsic cosmetic properties however remain quite poor, in particular due to the fact that the relatively aggressive nature of such a cleansing treatment may in the long term give rise to more or less pronounced damage on the hair fibres, associated in particular with the gradual removal of the fats or proteins contained in them or at their surface.

Thus, to improve the cosmetic properties of the above detergent compositions, and more particularly of those that are required to be applied to sensitized hair (i.e. hair that is generally damaged or embrittled by the action of external atmospheric agents such as light and bad weather, and/or mechanical or chemical treatments such as brushing, combing, dyeing, bleaching, permanent-waving and/or relaxing), it is now common practice to introduce into these compositions additional cosmetic agents known as conditioning agents, which are intended mainly to repair or limit the harmful or undesirable effects caused by the various treatments or attacking factors to which keratin materials, in particular keratin fibres and in particular the hair are more or less repeatedly subjected. These conditioning agents may, of course, also improve the cosmetic behaviour of natural hair.

It is thus known practice to use cationic polymers and silicones as conditioning agents in detergent cosmetic compositions such as shampoos, to give the hair satisfactory cosmetic properties, in particular in terms of sheen, softness, lightness, a natural feel and improved disentangling ability.

However, the use of these conditioning agents in cosmetic washing and conditioning compositions does not give the hair satisfactory and long-lasting styling properties. This is because the styling effects, such as the provision of form retention, of body and/or of manageability to the hair often remain insufficient and have a tendency to fade away after washing the hair with a standard shampoo. In point of fact, consumers are increasingly in search of washing compositions that are not only capable of appropriately conditioning the hair, but also capable of affording satisfactory and long-lasting styling effects.

In order to be able to meet these requirements, compositions intended for washing and conditioning the hair comprising organosilanes, such as 3-aminopropyltriethoxysilane, have been developed. Mention may in particular be made of patent application EP 2 343 039 which describes shampoos comprising anionic, amphoteric and non-ionic surfactants, silicones, cationic polymers and organosilanes, in particular 3-aminopropyltriethoxysilane. These clear and stable washing compositions make it possible to confer satisfactory styling effects, in particular volume and body, and also a soft feel, to the hair.

In particular, the applications FR 2 954 099, US 2011/158927 and the shampoos, commercialized by Franck Provost (XP-002763763) and Bern Estar (XP-002763764) disclosed cosmetic compositions comprising anionic surfactants and organosilanes. Further, the Silsoft A⁺ from Momentive is a known amino silicone comprising at least one polyoxyalkylenated group (XP-002763765).

However, it has been noted that these compositions do not make it possible to achieve optimal styling effects while at the same time having high conditioning performance levels, in particular in terms of softness, straightening and lightness, in particular on fine hair.

Moreover, the qualities regarding use of these compositions, such as for example the initiation, the abundance and the quality of the foam generated, do not yet give entire satisfaction.

There is thus a real need to develop clear cosmetic compositions intended for cleansing and conditioning keratin materials containing organosilanes which do not have all the drawbacks described above, and which make it possible to condition the hair satisfactorily while at the same time providing long-lasting styling effects.

The applicant has discovered, surprisingly, that it is possible to formulate detergent and conditioning compositions for keratin materials, which have the desired properties, by combining anionic surfactants, organosilanes and particular silicones.

Thus, a subject of the invention is a composition, in particular a cosmetic composition, in particular a cosmetic hair composition, comprising:
- at least one anionic surfactant chosen from polyoxyalkylenated (C₆-C₂₄)alkyl(amido) ether carboxylic acids and salts comprising from 2 to 50 ethylene oxide groups;
- at least one organosilane chosen from the compounds of formula (I) and/or oligomers thereof:

   R₁Si(OR₂)_{z}(R₃)ₓ(OH)_{y} (I)

   in which:
   - R₁ is a linear or branched, saturated C₁-C₂₂ hydrocarbon-based chain, which may be substituted with an amine group NH₂ or NHR (R ₌ C₁-C₂₀ alkyl),
   - R₂ represents an alkyl group comprising from 1 to 4 carbon atoms,
   - R₃ represents an alkyl group comprising from 1 to 4 carbon atoms,
   - y is 0,
   - z denotes an integer ranging from 1 to 3,
   - x denotes an integer ranging from 0 to 2,
   - with z + x + y = 3; and
- at least one amino silicone comprising at least one polyoxyalkylenated groupin a total amount ranging from 0.01 % to 5% by weight, relative to the total weight of the composition.

It has been noted that, with the compositions according to the invention, the hair, and more particularly fine hair, exhibits improved softness, suppleness and straightening; it disentangles easily and it remains light.

The hair also appears to be more coated, which is most particularly pleasing in the case of fine hair which then appears to be denser, healthy and in good condition. Moreover, the compositions in accordance with the present invention provide long-lasting styling effects, in particular in terms of providing volume, body and manageability, these being effects that become stronger with increased application. Furthermore, the compositions according to the invention make it possible to facilitate the shaping of the hair, in particular of fine hair.

Finally, the compositions according to the invention have optimal qualities regarding use, in particular in terms of foam initiation, foam abundance and quality, and foam persistence.

In addition, by virtue of the invention, it is possible to obtain clear compositions. The term "clear composition" is intended to mean a composition through which it is possible to see distinctly with the naked eye.

The clarity of the composition can be characterized by a measurement of turbidity according to the NTU method, using a 2100P turbidimeter from the company HACH, at ambient temperature (25°C). The turbidity of the compositions according to the invention is preferably less than 100 NTU units, preferentially less than 50 NTU units, in particular less than 20 NTU units and even more particularly less than 10 NTU units.

In the present description, the expression "at least one" is equivalent to the expression "one or more" and can substitute for said expression; the expression "between" is equivalent to the expression "ranging from" and can substitute for said expression, which implies that the limits are included.

### Anionic surfactants

The composition according to the invention comprises at least one anionic surfactant chosen from polyoxyalkylenated (C₆-C₂₄)alkyl(amido) ether carboxylic acids and salts comprising from 2 to 50 ethylene oxide groups.

The term "anionic surfactant" is intended to mean a surfactant comprising, as ionic or ionizable groups, only anionic groups.

In the present description, a species is termed as being "anionic" when it bears at least one permanent negative charge or when it can be ionized as a negatively charged species, under the conditions of use of the composition of the invention (for example the medium or the pH) and not comprising any cationic charge.

Among the above carboxylic surfactants, mention may be made most particularly of the compounds sold by the company Kao under the name Akypo.

The polyoxyalkylenated alkyl(amido) ether carboxylic acids that may be used are preferably chosen from those of formula (1):

R_{1'}-(OC₂H₄)_{n'}-OCH₂COOA (1)

in which:
- R_{1'} represents a linear or branched C₆-C₂₄ alkyl or alkenyl radical, a (C₈-C₉)alkylphenyl radical, an R_{2'}CONH-CH₂-CH₂- radical with R_{2'}, denoting a linear or branched C₉-C₂₁ alkyl or alkenyl radical; preferably R_{1'} is a C₈-C₂₀, preferably C₈-C₁₈, alkyl radical and aryl preferably denotes phenyl,
- n' is an integer or decimal number (average value) ranging from 2 to 24 and preferably from 2 to 10,
- A denotes H, ammonium, Na, K, Li, Mg or a monoethanolamine or triethanolamine residue.

It is also possible to use mixtures of compounds of formula (1), in particular mixtures of compounds containing different R_{1'} groups.

The polyoxyalkylenated alkyl(amido) ether carboxylic acids that are particularly preferred are those of formula (1) in which:
- R_{1'} denotes a linear or branched C₈-C₂₂, in particular C₁₀-C₁₆ or even C₁₂-C₁₄ alkyl radical, or alternatively a (C₈-C₉)alkylphenyl radical;
- A denotes a hydrogen or sodium atom, and
- n' ranges from 2 to 20, preferably from 2 to 10.

Even more preferentially, use is made of compounds of formula (1) in which R_{1'} denotes a C₁₂ alkyl radical, A denotes a hydrogen or sodium atom and n' ranges from 2 to 10.

When the anionic surfactant is in salt form, said salt may be chosen from alkali metal salts, such as the sodium or potassium salt, ammonium salts, amine salts and in particular amino alcohol salts, and alkaline-earth metal salts, such as the magnesium salt.

Examples of amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular the sodium or magnesium salts are preferably used.

Preferably, it comprises one or more anionic surfactants chosen from anionic surfactants of the type polyoxyalkylenated alkyl(amido) ether carboxylic acid of formula (1) above; particularly in acid form or in the form of alkali metal or alkaline-earth metal, ammonium or amino alcohol salts; preferentially in acid form.

In a second particular embodiment of the invention, the composition comprises:
- one or more carboxylic anionic surfactants of the type polyoxyalkylenated alkyl(amido) ether carboxylic acid of formula (1) above; particularly in acid form or in the form of alkali metal or alkaline-earth metal, ammonium or amino alcohol salts; and
- one or more sulfate anionic surfactants; preferably of C₆-C₂₄, in particular C₁₂-C₂₀, alkyl sulfate type, and/or of C₆-C₂₄, in particular C₁₂-C₂₀, alkyl ether sulfate type; preferably comprising from 2 to 20 ethylene oxide units; particularly in the form of alkali metal or alkaline-earth metal, ammonium or amino alcohol salts.

The anionic surfactant(s) are preferably present in the composition according to the invention in a total amount ranging from 1% to 35% by weight, in particular from 3% to 25% by weight and better still from 5% to 22% by weight relative to the total weight of the composition.

In one preferred embodiment, the composition according to the invention comprises at least one anionic surfactant of the type polyoxyalkylenated alkyl(amido) ether carboxylic acid of formula (1) above; particularly in acid form or in the form of alkali metal or alkaline-earth metal, ammonium or amino alcohol salts, in a content preferably ranging from 1% to 15% by weight, in particular from 1.5% 10% by weight, better still from 2% to 5% by weight relative to the total weight of the composition.

### Organosilanes

The composition according to the invention comprises at least one organosilane, chosen from the compounds of formula (I) and/or oligomers thereof:

R₁Si(OR₂)_{z}(R₃)ₓ(OH)_{y} (I)

in which
- R₁ is a linear or branched, preferably linear, saturated C₁-C₂₂, in particular C₂-C₁₂, hydrocarbon-based chain, which may be substituted with an amine group NH₂ or NHR (R = C₁-C₂₀, in particular C₁-C₆, alkyl),
- R₂ represents an alkyl group comprising from 1 to 4 carbon atoms, better still a linear alkyl group comprising from 1 to 4 carbon atoms, and preferably the methyl or ethyl group,
- R₃ represents an alkyl group comprising from 1 to 4 carbon atoms, better still a linear alkyl group comprising from 1 to 4 carbon atoms, and preferably the methyl or ethyl group,
- y is 0,
- z denotes an integer ranging from 1 to 3, and
- x denotes an integer ranging from 0 to 2,
with z + x + y = 3.

The term "oligomer" is intended to mean the polymerization products of the compounds having formula (I) including from 2 to 10 silicon atoms.

Preferentially, z = 3 and thus x = y = 0, or else z = 2, x = 1 and y = 0.

In one embodiment of the invention, R₁ represents a C₁-C₆, preferably C₂-C₄, amino alcohol group, z = 3 and x ₌ y ₌ 0.

Preferably, the composition according to the invention comprises one or more organosilanes chosen from octyltriethoxysilane (OTES), dodecyltriethoxysilane, octade-cyltriethoxysilane, hexadecyltriethoxysilane, 3-aminopropyltriethoxysilane (APTES), methyltriethoxysilane (MTES), 2-aminoethyltriethoxysilane (AETES), 3-aminopropylmethyldiethoxysilane, N-(2-aminoethyl)-3-aminopropyltriethoxysilane, 3-(m-aminophenoxy)propyltrimethoxysilane, p-aminophenyltrimethoxysilane, N-(2-aminoethylaminomethyl)phenethyltrimethoxysilane, and oligomers thereof; and more particularly chosen from 3-aminopropyltriethoxysilane (APTES) and oligomers thereof.

The organosilanes used in the composition of the invention, in particular those comprising a basic function, may be partially or totally neutralized in order to improve the water-solubility thereof. In particular, the neutralizing agent can be chosen from organic or mineral acids, more particularly from carboxylic organic acids, in particular those which are C₂-C₈, optionally comprising one or more hydroxyl functions. Mention may be made in particular of citric acid, tartaric acid, lactic acid, acetic acid and also hydrochloric acid.

When it is present, the concentration of the neutralizing agent, in particular of the acid, can range from 0.01% to 5% by weight, preferably from 0.05% to 3% by weight and better still from 0.1% to 2% by weight relative to the total weight of the composition.

Preferably, the optionally neutralized organosilanes according to the invention are water-soluble and in particular soluble at a concentration of 2%, better still at a concentration of 5% and even better still at a concentration of 10% by weight in water at a temperature of 25°C and at atmospheric pressure (1 atm). The term "soluble" is intended to mean the formation of a single macroscopic phase.

Preferably, the composition according to the invention comprises the organosilane(s) in an amount ranging from 0.01% to 10% by weight, preferably from 0.1% to 5% by weight and even better still from 0.2% to 1% by weight, relative to the total weight of the composition.

### Amino silicones

The composition according to the invention comprises at least one amino silicone, different from the organosilane(s) mentioned above, comprising at least one polyoxyalkylenated group.

The term "amino silicone" denotes any silicone comprising at least one primary, secondary or tertiary amine or a quaternary ammonium group.

The term "polyoxyalkylenated group" is intended to mean a group comprising at least two alkylene oxide units, in particular C₂-C₆, preferably C₂-C₄, better still C₂-C₃, alkylene oxide units. Preferably, the units constituting the polyoxyalkylenated group are chosen from ethylene oxide and/or propylene oxide units.

The weight-average molecular weights (Mw) of the amino silicones may be measured by gel permeation chromatography (GPC) at ambient temperature (25°C), as polystyrene equivalent. The columns used are µ styragel columns. The eluent is THF and the flow rate is 1 ml/min. 200 µl of a 0.5% by weight solution of silicone in THF are injected. Detection is performed by refractometry and UV-metry.

Preferably, the amino silicone(s) that may be used in the context of the invention are chosen from, alone or as a mixture:
- (b) the polyoxyalkylenated amino silicones, in particular of multiblock type (AB)n, A being a polysiloxane block and B being a polyoxyalkylene block, and comprising at least one amine group.

Said silicones are preferably constituted of repeating units of formula (111.1) and/or of formula (III.2) below:

[-(SiMe₂O)ᵥ-SiMe₂-R₇-N(R₈)-R₉-O(C₃H₆O)ₐ(C₂H₄O)_{b}-R₉-N(R₈)-R₇-] (III.1)

[-(SiMe₂O)ᵥ-SiMe₂-R₇-N(R₈)-R₉-O(C₃H₆O)ₐ(C₂H₄O)_{b}-] (III.2)

in which:
- a is an integer greater than or equal to 1, preferably ranging from 5 to 200, or even from 60 to 100, and more particularly ranging from 10 to 100;
- b is an integer between 0 and 200, preferably ranging from 4 to 100, or even from 5 to 50, and more particularly between 5 and 30;
- v is an integer ranging from 1 to 10 000 and more particularly from 10 to 5000;
- R₈, which may be identical or different, are a hydrogen atom or a methyl, preferably methyl;
- R₇, which may be identical or different, represent a linear or branched divalent C₂-C₁₂ hydrocarbon-based radical, optionally comprising one or more heteroatoms such as oxygen; preferably, R₇ denotes an ethylene radical, a linear or branched propylene radical, a linear or branched butylene radical or a CH₂CH₂CH₂OCH₂CH(OH)CH₂- radical; preferentially, R₇ represents a CH₂CH₂CH₂OCH₂CH(OH)CH₂- radical;
- R₉, which may be identical or different, represent a linear or branched C₂-C₁₂ divalent hydrocarbon-based radical, optionally comprising one or more heteroatoms such as oxygen; preferably, R₉ denotes an ethylene radical, a linear or branched propylene radical, a linear or branched butylene radical, or a CH₂CH₂CH₂OCH₂CH(OH)CH₂- radical; preferentially, R₉ denotes -CH(CH₃)-CH₂-.

Preferably, the siloxane blocks -(SiMe₂O)ᵥ- represent 50 mol% to 95 mol% of the total weight of the silicone, more particularly 70 mol% to 85 mol%.

The amine content is preferably between 0.02 and 0.5 meq/g of silicone in a 30% solution in dipropylene glycol, more particularly between 0.05 and 0.2 meq/g.

The weight-average molecular weight (Mw) of the silicone is preferably between 5000 and 1 000 000 and more particularly between 10 000 and 200 000.

Preferentially, the amino silicones are chosen form the polyoxyalkenated amino silicones, and in particular from the amino silicones constituted at least of repeating units of general formula (III.2).

Among the polyoxyalkylenated amino silicones, mention may particularly made of the compounds having the INCI name PEG-40/PPG-8 methylaminopropyl/hydroxypropyl dimethicone copolymer such as the compound provided by the company Momentive Performance Materials under the trade name Silsoft A+.

Mention may also be made of the compounds having the INCI name Bisamino PEG/PPG-41/3 aminoethyl PG-propyl dimethicone such as the compound provided by the company Momentive Performance Materials under the trade name Silsoft A843.

Mention may also be made of the compounds having the INCI name Methoxy PEG/PPG-7/3 aminopropyl dimethicone such as the product provided by the company Evonik under the name AbilSoft AF100.

Preferentially, the amino silicones are chosen from the compounds having the INCI name PEG-40/PPG-8 methylaminopropyl/hydroxypropyl dimethicone copolymer, Bisamino PEG/PPG-41/3 aminoethyl PG-propyl dimethicone.

The composition according to the invention comprises said amino silicone(s) in a total amount ranging from 0.01% to 5% by weight relative to the total weight of the composition preferably in an amount ranging from 0.05% to 2% by weight and preferentially from 0.1% to 0.5% by weight, relative to the total weight of the composition.

### Amphoteric surfactants

The composition according to the invention may also comprise one or more amphoteric surfactants.

The amphoteric surfactants that may be used in the invention may be optionally quaternized secondary or tertiary aliphatic amine derivatives, in which the aliphatic radical is a linear or branched chain comprising from 8 to 22 carbon atoms, said amine derivatives containing at least one hydrosolubilizing anionic group, for instance a carboxylate, sulfonate, sulfate, phosphate or phosphonate group.

Mention may be made in particular of (C₈-C₂₀)alkylbetaines, sulfobetaines, (C₈-C₂₀)alkylsulfobetaines, (C₈-C₂₀)alkylamido(C₁-C₆)alkylbetaines, such as cocami-dopropylbetaine, (C₈-C₂₀)alkylamido(C₁-C₆)alkylsulfobetaines, and also mixtures thereof.

Among the derivatives of optionally quaternized secondary or tertiary aliphatic amines that may be used, mention may also be made of the products having respective structures (IV.1) and (IV.2) below:

Rₐ-CON(Z)CH₂-(CH₂)ₘ-N⁺(R_{b})(R_{c})(CH₂COO⁻) (IV. 1)

in which:
- Rₐ represents a C₁₀-C₃₀ alkyl or alkenyl group derived from an acid Rₐ-COOH preferably present in hydrolysed coconut oil, or a heptyl, nonyl or undecyl group,
- R_{b} represents a β-hydroxyethyl group,
- R_{c} represents a carboxymethyl group;
group are chosen from ethylene oxide and/or propylene oxide units.

The weight-average molecular weights (Mw) of the amino silicones may be measured by gel permeation chromatography (GPC) at ambient temperature (25°C), as polystyrene equivalent. The columns used are µ styragel columns. The eluent is THF and the flow rate is 1 ml/min. 200 µl of a 0.5% by weight solution of silicone in THF are injected. Detection is performed by refractometry and UV-metry.

Preferably, the amino silicone(s) that may be used in the context of the invention are chosen from, alone or as a mixture:
- (b) the polyoxyalkylenated amino silicones, in particular of multiblock type (AB)n, A being a polysiloxane block and B being a polyoxyalkylene block, and comprising at least one amine group.

Said silicones are preferably constituted of repeating units of formula (111.1) and/or of formula (III.2) below:

[-(SiMe₂O)ᵥ-SiMe₂-R₇-N(R₈)-R₉-O(C₃H₆O)ₐ(C₂H₄O)_{b}-R₉-N(R₈)-R₇-] (III.1)

[-(SiMe₂O)ᵥ-SiMe₂-R₇-N(R₈)-R₉-O(C₃H₆O)ₐ(C₂H₄O)_{b}-] (III.2)

in which:
- a is an integer greater than or equal to 1, preferably ranging from 5 to 200, or even from 60 to 100, and more particularly ranging from 10 to 100;
- b is an integer between 0 and 200, preferably ranging from 4 to 100, or even from 5 to 50, and more particularly between 5 and 30;
- v is an integer ranging from 1 to 10 000 and more particularly from 10 to 5000;
- R₈, which may be identical or different, are a hydrogen atom or a methyl, preferably methyl;
- R₇, which may be identical or different, represent a linear or branched divalent C₂-C₁₂ hydrocarbon-based radical, optionally comprising one or more heteroatoms such as oxygen; preferably, R₇ denotes an ethylene radical, a linear or branched propylene radical, a linear or branched butylene radical or a CH₂CH₂CH₂OCH₂CH(OH)CH₂- radical; preferentially, R₇ represents a CH₂CH₂CH₂OCH₂CH(OH)CH₂- radical;
- R₉, which may be identical or different, represent a linear or branched C₂-C₁₂ divalent hydrocarbon-based radical, optionally comprising one or more heteroatoms such as oxygen; preferably, R₉ denotes an ethylene radical, a linear or branched propylene radical, a linear or branched butylene radical, or a CH₂CH₂CH₂OCH₂CH(OH)CH₂- radical; preferentially, R₉ denotes -CH(CH₃)-CH₂-.

Preferably, the siloxane blocks -(SiMe₂O)ᵥ- represent 50 mol% to 95 mol% of the total weight of the silicone, more particularly 70 mol% to 85 mol%.

The amine content is preferably between 0.02 and 0.5 meq/g of silicone in a 30% solution in dipropylene glycol, more particularly between 0.05 and 0.2 meq/g.

The weight-average molecular weight (Mw) of the silicone is preferably between 5000 and 1 000 000 and more particularly between 10 000 and 200 000.

Preferentially, the amino silicones are chosen form the polyoxyalkenated amino silicones, and in particular from the amino silicones constituted at least of repeating units of general formula (III.2).

Among the polyoxyalkylenated amino silicones, mention may particularly made of the compounds having the INCI name PEG-40/PPG-8 methylaminopropyl/hydroxypropyl dimethicone copolymer such as the compound provided by the company Momentive Performance Materials under the trade name Silsoft A+.

Mention may also be made of the compounds having the INCI name Bisamino PEG/PPG-41/3 aminoethyl PG-propyl dimethicone such as the compound provided by the company Momentive Performance Materials under the trade name Silsoft A843.

Mention may also be made of the compounds having the INCI name Methoxy PEG/PPG-7/3 aminopropyl dimethicone such as the product provided by the company Evonik under the name AbilSoft AF100.

Preferentially, the amino silicones are chosen from the compounds having the IN-CI name PEG-40/PPG-8 methylaminopropyl/hydroxypropyl dimethicone copolymer, Bisamino PEG/PPG-41/3 aminoethyl PG-propyl dimethicone or Quaternium-80.

The composition according to the invention comprises said amino silicone(s) in a total amount ranging from 0.01% to 5% by weight relative to the total weight of the composition preferably in an amount ranging from 0.05% to 2% by weight and preferentially from 0.1% to 0.5% by weight, relative to the total weight of the composition.

### Amphoteric surfactants

The composition according to the invention may also comprise one or more amphoteric surfactants.

The amphoteric surfactants that may be used in the invention may be optionally quaternized secondary or tertiary aliphatic amine derivatives, in which the aliphatic radical is a linear or branched chain comprising from 8 to 22 carbon atoms, said amine derivatives containing at least one hydrosolubilizing anionic group, for instance a carboxylate, sulfonate, sulfate, phosphate or phosphonate group.

Mention may be made in particular of (C₈-C₂₀)alkylbetaines, sulfobetaines, (C₈-C₂₀)alkylsulfobetaines, (C₈-C₂₀)alkylamido(C₁-C₆)alkylbetaines, such as cocami-dopropylbetaine, (C₈-C₂₀)alkylamido(C₁-C₆)alkylsulfobetaines, and also mixtures thereof.

Among the derivatives of optionally quaternized secondary or tertiary aliphatic amines that may be used, mention may also be made of the products having respective structures (IV.1) and (IV.2) below:

Rₐ-CON(Z)CH₂-(CH₂)ₘ-N⁺(R_{b})(R_{c})(CH₂COO⁻) (IV.1)

in which:
- Rₐ represents a C₁₀-C₃₀ alkyl or alkenyl group derived from an acid Rₐ-COOH preferably present in hydrolysed coconut oil, or a heptyl, nonyl or undecyl group,
- R_{b} represents a β-hydroxyethyl group,
- R_{c} represents a carboxymethyl group;
- m is equal to 0, 1 or 2,
- Z represents a hydrogen atom or a hydroxyethyl or carboxymethyl group,

R_{a'}-CON(Z)CH₂-(CH₂)_{m'}-N(R_{b'})(R_{c'}) (IV.2)

in which:
- R_{b'} represents -CH₂CH₂OX', with X' representing -CH₂-COOH, CH₂-COOZ', - CH₂CH₂-COOH, -CH₂CH₂-COOZ', or a hydrogen atom,
- R_{c'} represents -(CH₂)_{z2}-Y', with z2 = 1 or 2, and Y' representing -COOH, -COOZ',
- CH₂-CHOH-SO₃H or CH₂-CHOH-SO₃Z',
- Z' represents an ion derived from an alkali metal or alkaline-earth metal, such as sodium, potassium or magnesium; an ammonium ion; or an ion derived from an organic amine and in particular from an amino alcohol, such as monoethanolamine, diethanolamine or triethanolamine, monoisopropanolamine, diisopropanolamine or triisopropanolamine, 2-amino-2-methyl-1-propanol, 2-amino-2-methyl-1,3-propanediol and tris(hydroxymethyl)aminomethane;
- m' is equal to 0, 1 or 2,
- Z represents a hydrogen atom or a hydroxyethyl or carboxymethyl group,
- R_{a'} represents a C₁₀-C₃₀ alkyl or alkenyl group of an acid R_{a'}COOH preferably present in hydrolysed linseed oil or coconut oil, an alkyl group, in particular a C₁₇ alkyl group, and its iso form, or an unsaturated C₁₇ group.

The compounds corresponding to formula (IV.2) are particularly preferred.

Among the compounds of formula (IV.2) for which X' represents a hydrogen atom, mention may be made of the compounds known under the (CTFA) names sodium cocoamphoacetate, sodium lauroamphoacetate, sodium caproamphoacetate and sodium capryloamphoacetate.

Other compounds of formula (IV.2) are known under the (CTFA) names disodium cocoamphodiacetate, disodium lauroamphodiacetate, disodium caproamphodiacetate, disodium capryloamphodiacetate, disodium cocoamphodipropionate, disodium lauroamphodipropionate, disodium caproamphodipropionate, disodium capryloamphodipropionate, lauroamphodipropionic acid and cocoamphodipropionic acid.

As examples of compounds of formula (IV.2), mention may be made of the cocoamphodiacetate sold by the company Rhodia under the trade name Miranol^{®} C2M Concentrate, the sodium cocoamphoacetate sold under the trade name Miranol Ultra C 32 and the product sold by the company Chimex under the trade name Chimexane HA.

Use may also be made of the compounds of formula (IV.3):

R_{a"}-NH-CH(Y")-(CH₂)_{q}-C(O)-NH-(CH₂)_{q'}-N(R_{b"})(R_{c"}) (IV.3)

in which:
- R_{a"} represents a C₁₀-C₃₀ alkyl or alkenyl group of an acid R_{a"}-C(O)OH, which is preferably present in hydrolyzed linseed oil or coconut oil;
- Y" represents the group -C(O)OH, -C(O)OZ", -CH₂-CH(OH)-SO₃H or the group CH₂-CH(OH)-SO₃-Z", with Z" representing a cation resulting from an alkali metal or alkaline-earth metal, such as sodium, an ammonium ion or an ion resulting from an organic amine;
- R_{b"} and R_{c"}, independently of one another, represent a C₁-C₄ alkyl or hydroxyalkyl radical; and
- q and q' denote, independently one another, an integer ranging from 1 to 3.

Among the compounds of formula (IV.3), mention may in particular be made of the compound classified in the CTFA dictionary under the name sodium diethylaminopropyl cocoaspartamide and in particular the product sold by the company Chimex under the name Chimexane HB.

Preferably, the amphoteric surfactants are chosen from (C₈-C₂₀)alkylbetaines, (C₈-C₂₀)alkylamido(C₁-C₆)alkylbetaines, (C₈-C₂₀)alkylamphoacetates and (C₈-C₂₀)alkylamphodiacetates, and mixtures thereof, and in particular from cocobetaine and cocoamidopropylbetaine.

Preferably, when they are present, the composition according to the invention comprises said amphoteric surfactant(s) in an amount ranging from 1% to 15% by weight, in particular ranging from 2% to 10% by weight, preferentially from 3% to 8% by weight, relative to the total weight of the composition.

### Cationic polymers

The composition according to the invention may also comprise one or more cationic polymers.

The term "cationic polymer" is intended to mean any polymer comprising cationic groups and/or groups that can be ionized to cationic groups. Preferably, the cationic polymer is hydrophilic or aphiphilic. The preferred cationic polymers are chosen from those that contain units comprising primary, secondary, tertiary and/or quaternary amine groups that may either form part of the main polymer chain or may be borne by a side substituent directly connected thereto.

The cationic polymers preferably have a weight-average molar mass (Mw) of between 500 and 5×10⁶ approximately and preferably between 10³ and 3×10⁶ approximately.

Among the cationic polymers, mention may be made more particularly of:
a₁) homopolymers or copolymers derived from acrylic or methacrylic esters or amides and comprising at least one of the units of formula (V.1) below: 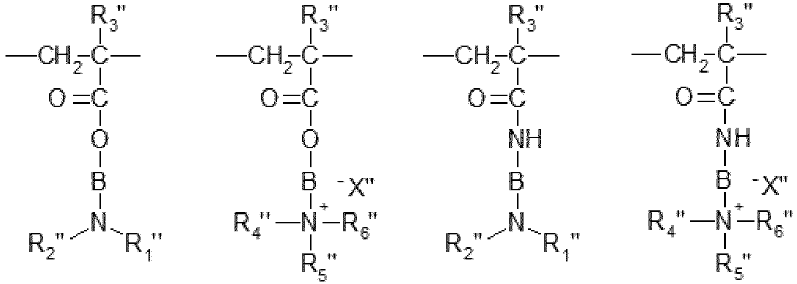 in which:
- R₃^{"}, which may be identical or different, denote a hydrogen atom or a CH₃ radical;
- B, which may be identical or different, represent a linear or branched divalent alkyl group of 1 to 6 carbon atoms, preferably 2 or 3 carbon atoms, or a hydroxyalkyl group of 1 to 4 carbon atoms;
- R₄", R₅^{"} and R₆^{"}, which may be identical or different, represent an alkyl group containing from 1 to 18 carbon atoms or a benzyl radical, preferably an alkyl group containing from 1 to 6 carbon atoms;
- R₁" and R₂", which may be identical or different, represent a hydrogen atom or an alkyl group containing from 1 to 6 carbon atoms, and preferably methyl or ethyl;
- X"⁻ denotes an anion derived from a mineral or organic acid, such as a methosulfate anion or a halide such as chloride or bromide.

The copolymers of family (V.1) may also contain one or more units derived from comonomers which may be chosen from the family of acrylamides, methacrylamides, diacetone acrylamides, acrylamides and methacrylamides substituted on the nitrogen with lower (C₁-C₄) alkyls, acrylic or methacrylic acids or esters thereof, vinyllactams such as vinylpyrrolidone or vinylcaprolactam, and vinyl esters.

Among these copolymers of family (V.1), mention may be made of:
- copolymers of acrylamide and of dimethylaminoethyl methacrylate quaternized with dimethyl sulfate or with a dimethyl halide, such as that sold under the name Hercofloc by the company Hercules,
- copolymers of acrylamide and of methacryloyloxyethyltrimethylammonium chloride, such as those sold under the name Bina Quat P 100 by the company Ciba Geigy,
- the copolymer of acrylamide and of methacryloyloxyethyltrimethylammonium methosulfate, such as that sold under the name Reten by the company Hercules,
- quaternized or non-quaternized vinylpyrrolidone/dialkylaminoalkyl acrylate or methacrylate copolymers, such as the products sold under the name Gafquat by the company ISP, for instance Gafquat 734 or Gafquat 755, or alternatively the products known as Copolymer 845, 958 and 937. These polymers are described in detail in French patents 2 077 143 and 2 393 573;
- dimethylaminoethyl methacrylate/vinylcaprolactam/vinylpyrrolidone terpolymers, such as the product sold under the name Gaffix VC 713 by the company ISP,
- vinylpyrrolidone/methacrylamidopropyldimethylamine copolymers, such as the those sold under the name Styleze CC 10 by ISP,
- quaternized vinylpyrrolidone/dimethylaminopropylmethacrylamide copolymers such as the product sold under the name Gafquat HS 100 by the company ISP,
- preferably crosslinked polymers of methacryloyloxy(C₁-C₄)alkyltri(C₁-C₄)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homopolymerization or copolymerization being followed by crosslinking with an olefinically unsaturated compound, in particular methylenebisacrylamide. Use may be made more particularly of a crosslinked acrylamide/methacryloyloxyethyltrimethylammonium chloride copolymer (20/80 by weight) in the form of a dispersion comprising 50% by weight of said copolymer in mineral oil. This dispersion is sold under the name Salcare^{®} SC 92 by the company Ciba. Use may also be made of a crosslinked methacryloyloxyethyltrimethylammonium chloride homopolymer comprising approximately 50% by weight of the homopolymer in mineral oil or in a liquid ester. These dispersions are sold under the names Salcare^{®} SC 95 and Salcare^{®} SC 96 by the company Ciba;
a₂) cationic polysaccharides, in particular cationic celluloses and galactomannan gums. Among the cationic polysaccharides, mention may be made more particularly of cellulose ether derivatives comprising quaternary ammonium groups, cationic cellulose copolymers or cellulose derivatives grafted with a water-soluble quaternary ammonium monomer and cationic galactomannan gums.

The cellulose ether derivatives comprising quaternary ammonium groups are in particular described in FR 1 492 597, and mention may be made of the polymers sold under the name Ucare Polymer JR (JR 400 LT, JR 125 and JR 30M) or LR (LR 400 and LR 30M) by the company Amerchol. These polymers are also defined in the CTFA dictionary as quaternary ammoniums of hydroxyethylcellulose that have reacted with an epoxide substituted with a trimethylammonium group.

Cationic cellulose copolymers or cellulose derivatives grafted with a water-soluble quaternary ammonium monomer are described in particular in patent US 4 131 576, and mention may be made of hydroxyalkyl celluloses, for instance hydroxymethyl-, hydroxyethyl- or hydroxypropylcelluloses grafted, in particular, with a methacryloylethyltrimethylammonium, methacrylamidopropyltrimethylammonium or dimethyldiallylammonium salt. The commercial products corresponding to this definition are more particularly the products sold under the names Celquat L 200 and Celquat H 100 by the company National Starch.

The cationic galactomannan gums are described more particularly in patents US 3 589 578 and US 4 031 307, and mention may be made of guar gums comprising cationic trialkylammonium groups. Use is made, for example, of guar gums modified with a 2,3-epoxypropyltrimethylammonium salt (for example, chloride). Such products are in particular sold under the names Jaguar C13 S, Jaguar C 15, Jaguar C 17 and Jaguar C162 by the company Rhodia;
a₃) polymers formed from piperazinyl units and divalent alkylene or hydroxyalkylene radicals containing linear or branched chains, optionally interrupted with oxygen, sulfur or nitrogen atoms or with aromatic or heterocyclic rings, and also the oxidation and/or quaternization products of these polymers;
a₄) water-soluble polyamino amides prepared in particular by polycondensation of an acidic compound with a polyamine; these polyamino amides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyamino amide; these polyamino amides can be alkylated or, if they contain one or more tertiary amine functions, they can be quaternized;
a₅) polyaminoamide derivatives resulting from the condensation of polyalkylene polyamines with polycarboxylic acids followed by alkylation with difunctional agents. Mention may be made, for example, of adipic acid/dialkylaminohydroxyalkyldialkylenetriamine polymers in which the alkyl radical comprises from 1 to 4 carbon atoms and preferably denotes methyl, ethyl or propyl. Among these derivatives, mention may be made more particularly of the adipic acid/dimethylaminohydroxypropyl/diethylenetriamine polymers sold under the name Cartaretine F, F4 or F8 by the company Sandoz;
a₆) polymers obtained by reacting a polyalkylene polyamine comprising two primary amine groups and at least one secondary amine group with a dicarboxylic acid chosen from diglycolic acid and saturated aliphatic dicarboxylic acids containing from 3 to 8 carbon atoms; the mole ratio between the polyalkylene polyamine and the dicarboxylic acid preferably being between 0.8:1 and 1.4:1; the resulting polyamino amide being reacted with epichlorohydrin in a mole ratio of epichlorohydrin to the secondary amine group of the polyaminoamide preferably of between 0.5:1 and 1.8:1. Polymers of this type are sold in particular under the name Hercosett 57 by the company Hercules Inc. or else under the name PD 170 or Delsette 101 by the company Hercules in the case of the adipic acid/epoxypropyl/diethylenetriamine copolymer;
a₇) alkyldiallylamine or dialkyldiallylammonium cyclopolymers, such as homopolymers or copolymers comprising, as the main chain constituent, units corresponding to formula (V.2) or (V.3): 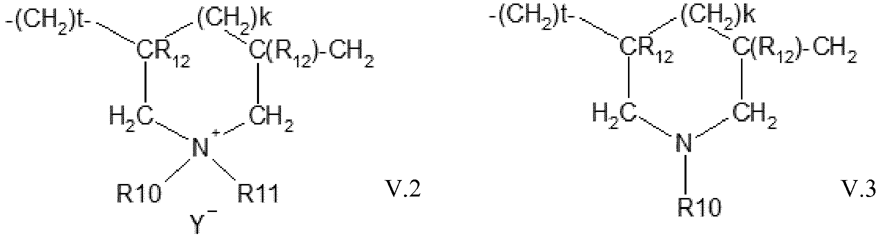 in which
   k and t are equal to 0 or 1, the sum k + t being equal to 1;
   R₁₂ denotes a hydrogen atom or a methyl radical;
   R₁₀ and R₁₁, independently one other, denote a C₁-C₆ alkyl group, a C₁-C₅ hydroxyalkyl group, a C₁-C₄ amidoalkyl group; or alternatively R₁₀ and R₁₁ may denote, together with the nitrogen atom to which they are attached, a heterocyclic group such as piperidyl or morpholinyl; R₁₀ and R₁₁, independently of one another, preferably denote a C₁-C₄ alkyl group;
   Y⁻ is an anion such as bromide, chloride, acetate, borate, citrate, tartrate, bisulfate, bisulfite, sulfate or phosphate.

Mention may be made more particularly of the dimethyldiallylammonium salt (for example chloride) homopolymer for example sold under the name Merquat 100 by the company Nalco and the copolymers of diallyldimethylammonium salt (for example chloride) and of acrylamide, sold in particular under the name Merquat 550 or Merquat 7SPR;
a₈) quaternary diammonium polymers comprising repeating units of formula (V.4): 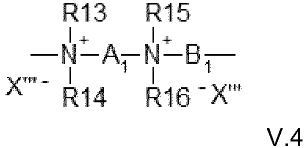 in which:
- R₁₃, R₁₄, R₁₅ and R₁₆, which may be identical or different, represent aliphatic, alicyclic or arylaliphatic radicals comprising from 1 to 20 carbon atoms or C₁-C₁₂ hydroxyalkyl aliphatic radicals,
   or else R₁₃, R₁₄, R₁₅ and R₁₆, together or separately, form, with the nitrogen atoms to which they are attached, heterocycles optionally comprising a second non-nitrogen heteroatom;
   or else R₁₃, R₁₄, R₁₅ and R₁₆ represent a linear or branched C₁-C₆ alkyl radical substituted with a nitrile, ester, acyl, amide or -CO-O-R₁₇-D or -CO-NH-R₁₇-D group, where R₁₇ is an alkylene and D is a quaternary ammonium group;
- A₁ and B₁ represent linear or branched, saturated or unsaturated, divalent polymethylene groups comprising from 2 to 20 carbon atoms, which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and
- X‴⁻ denotes an anion derived from a mineral or organic acid;
   it being understood that A₁, R₁₃ and R₁₅ can form, with the two nitrogen atoms to which they are attached, a piperazine ring;
   in addition, if A₁ denotes a linear or branched, saturated or unsaturated alkylene or hydroxyalkylene radical, B₁ may also denote a group (CH₂)ₙ₂-CO-D-OC-(CH₂)ₚ-with n₂ and p, which may be identical or different, being integers ranging from 2 to 20, and D denoting:
- a glycol residue of formula -O-V-O-, in which V denotes a linear or branched hydrocarbon-based radical, or a group corresponding to one of the following formulae: -(CH₂-CH₂-O)_{x'}-CH₂-CH₂- and -[CH₂-CH(CH₃)-O]_{y'}-CH₂-CH(CH₃)-, where x' and y' denote an integer from 1 to 4, representing a defined and unique degree of polymerization or any number from 1 to 4 representing an average degree of polymerization;
- a bis-secondary diamine residue such as a piperazine derivative;
- a bis-primary diamine residue of formula -NH-W-NH-, in which W denotes a linear or branched hydrocarbon-based radical, or else the divalent radical -CH₂-CH₂-S-S-CH₂-CH₂-;
- a ureylene group of formula -NH-CO-NH-.

Preferably, X‴⁻ is an anion, such as chloride or bromide. These polymers have a number-average molar mass (Mn) generally of between 1000 and 100 000.

Mention may be made more particularly of polymers that are composed of repeating units corresponding to the formula (V.5): 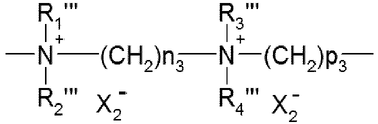
in which R₁^{‴}, R₂^{‴}, R₃^{‴} and R₄^{‴} which may be identical or different, denote an alkyl or hydroxyalkyl radical containing from 1 to 4 carbon atoms, n₃ and p₃ are integers ranging from 2 to 20, and X₂⁻ is an anion derived from a mineral or organic acid. A compound of formula (V.5) that is particularly preferred is the one for which R_{1‴}, R_{2‴}, R_{3‴} and R_{4‴} represent a methyl radical and n = 3, p = 6 and X₂ = Cl, which is known as Hexadimethrine chloride according to the INCI (CTFA) nomenclature;
a₉) polyquaternary ammonium polymers comprising units of formula (V.6): 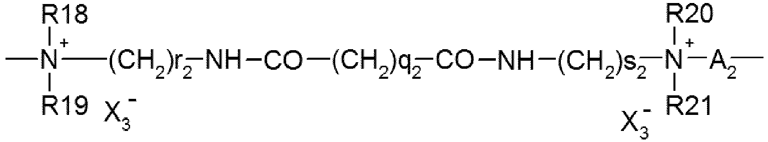 in which:
   R₁₃, R₁₉, R₂₀ and R₂₁, which may be identical or different, represent a hydrogen atom or a methyl, ethyl, propyl, β-hydroxyethyl, β-hydroxypropyl or - CH₂CH₂(OCH₂CH₂)ₚ₄OH radical, where p₄ is equal to 0 or to an integer of between 1 and 6, with the proviso that R₁₃, R₁₉, R₂₀ and R₂₁ do not simultaneously represent a hydrogen atom,
   r₂ and s₂, which may be identical or different, are integers between 1 and 6,
   q₂ is equal to 0 or to an integer between 1 and 34,
   X₃⁻ denotes an anion such as a halide,
   A₂ denotes a divalent radical of a dihalide or preferably represents -CH₂-CH₂-O-CH₂-CH₂-.
   Examples that may be mentioned include the products Mirapol^{®} A 15, Mirapol^{®} AD1, Mirapol^{®} AZ1 and Mirapol^{®} 175 sold by the company Miranol;
a₁₀) quaternary polymers of vinylpyrrolidone and of vinylimidazole, for instance the products sold under the names Luviquat^{®} FC 905, FC 550 and FC 370 by the company BASF;
a₁₁) polyamines such as Polyquart^{®} H sold by Cognis, referred to under the name Polyethylene glycol (15) tallow polyamine in the CTFA dictionary;
a₁₂) polymers comprising in their structure:
   - one or more units corresponding to formula (V.7) below: 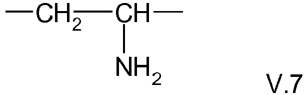
   - optionally one or more units corresponding to formula (V.8) below: 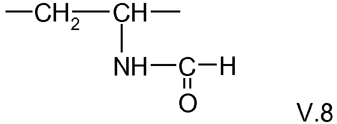
   In other words, these polymers may be chosen in particular from homopolymers or copolymers comprising one or more units derived from vinylamine and optionally one or more units derived from vinylformamide.

Preferably, these cationic polymers are chosen from polymers comprising, in their structure, from 5 mol% to 100 mol% of units corresponding to the formula (V.7) and from 0 mol% to 95 mol% of units corresponding to the formula (V.8), preferably from 10 mol% to 100 mol% of units corresponding to the formula (V.7) and from 0 mol% to 90 mol% of units corresponding to the formula (V.8).

These polymers may be obtained, for example, by partial hydrolysis of polyvinyl-formamide. This hydrolysis may take place in acidic or basic medium.

The weight-average molecular weight of said polymer, measured by light scattering, may range from 1000 to 3 000 000 g/mol, preferably from 10 000 to 1 000 000 and more particularly from 100 000 to 500 000 g/mol.

The polymers comprising units of formula (V.7) and optionally units of formula (V.8) are sold in particular under the name Lupamin by the company BASF, such as, for example, and in a non-limiting way, the products provided under the names Lupamin 9095, Lupamin 5095, Lupamin 1095, Lupamin 9030 (or Luviquat 9030) and Lupamin 9010.

Other cationic polymers which can be used in the context of the invention are cationic proteins or cationic protein hydrolysates, polyalkyleneimines, in particular polyethyleneimines, polymers comprising vinylpyridine or vinylpyridinium units, condensates of polyamines and of epichlorohydrin, quaternary polyureylenes and chitin derivatives.

Preferably, the cationic polymers are chosen from those of families a₁), a₂), a₇) et a₁₀) mentioned above.

Among the cationic polymers mentioned above, the ones that may preferably be used are cationic polysaccharides, in particular cationic celluloses and cationic galactomannan gums, and in particular quaternary cellulose ether derivatives such as the products sold under the name JR 400 by the company Amerchol, cationic cyclopolymers, in particular dimethyldiallylammonium salt (for example chloride) homopolymers or copolymers, sold under the names Merquat 100, Merquat 550 and Merquat S by the company Nalco, quaternary polymers of vinylpyrrolidone and of vinylimidazole, optionally crosslinked homopolymers or copolymers of methacryloyloxy(C₁-C₄)alkyltri(C₁-C₄)alkylammonium salts, and mixtures thereof.

In a particular embodiment, the cationic polymers which are likely to be used are chosen from the associative cationic polymers.

Among the cationic associative polymers, mention may be made of, alone or as a mixture:
- (A) cationic associative polyurethanes, which can be represented by general formula (Ia) below:

   R-X-(P)n-[L-(Y)m]r-L'-(P')p-X'-R'

   in which:
   R and R', which may be identical or different, represent a hydrophobic group or a hydrogen atom;
   X and X', which may be identical or different, represent a group comprising an amine function optionally bearing a hydrophobic group, or alternatively the group L";
   L, L' and L", which may be identical or different, represent a group derived from a diisocyanate;
   P and P', which may be identical or different, represent a group comprising an amine function optionally bearing a hydrophobic group;
   Y represents a hydrophilic group;
   r is an integer between 1 and 100 inclusive, preferably between 1 and 50 inclusive and in particular between 1 and 25 inclusive;
   n, m and p are each, independently of one another, between 0 and 1000 inclusive; the molecule containing at least one protonated or quaternized amine function and at least one hydrophobic group.

Preferably, the only hydrophobic groups are the groups R and R' at the chain ends.

One preferred family of cationic associative polyurethanes is the one corresponding to formula (Ia) described above in which:
R and R' both independently represent a hydrophobic group,
X and X' each represent a group L",
n and p are integers which are inclusively between 1 and 1000 and L, L', L", P, P',
Y and m have the meaning indicated above.

Another preferred family of cationic associative polyurethanes is the one corresponding to formula (Ia) above in which:
- n=p = 0 (the polymers do not comprise units derived from a monomer containing an amine function, incorporated into the polymer during the polycondensation),
- the protonated amine functions result from the hydrolysis of excess isocyanate functions, at the chain end, followed by alkylation of the primary amine functions formed with alkylating agents containing a hydrophobic group, i.e. compounds of the type RQ or R'Q, in which R and R' are as defined above and Q denotes a leaving group such as a halide or a sulfate, etc.

Yet another preferred family of cationic associative polyurethanes is the one corresponding to formula (Ia) above in which:
R and R' both independently represent a hydrophobic group,
X and X' both independently represent a group comprising a quaternary amine,
n = p = 0, and
L, L', Y and m have the meaning indicated above.

The number-average molecular weight (Mn) of the cationic associative polyurethanes is preferably between 400 and 500 000, in particular between 1000 and 400 000 inclusive and ideally between 1000 and 300 000 inclusive.

The expression "hydrophobic group" is intended to mean a radical or polymer containing a saturated or unsaturated, linear or branched hydrocarbon-based chain, which may contain one or more heteroatoms such as P, O, N or S, or a radical containing a perfluoro or silicone chain. When it denotes a hydrocarbon-based radical, the hydrophobic group comprises at least 10 carbon atoms, preferably from 10 to 30 carbon atoms, in particular from 12 to 30 carbon atoms and more preferentially from 18 to 30 carbon atoms.

Preferentially, the hydrocarbon-based group is derived from a monofunctional compound.

By way of example, the hydrophobic group may be derived from a fatty alcohol such as stearyl alcohol, dodecyl alcohol or decyl alcohol. It may also denote a hydrocarbon-based polymer, such as, for example, polybutadiene.

When X and/or X' denote(s) a group comprising a tertiary or quaternary amine, X and/or X' may represent one of the following formulae: 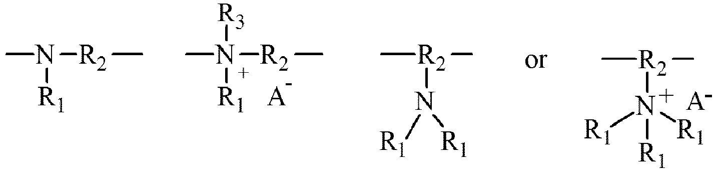 for X 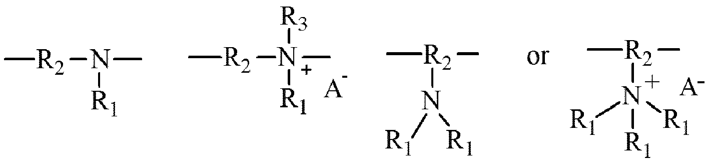 for X'
in which:
R₂ represents a linear or branched alkylene radical containing from 1 to 20 carbon atoms, optionally comprising a saturated or unsaturated ring, or an arylene radical,
one or more of the carbon atoms possibly being replaced with a heteroatom chosen from N, S, O and P;
R₁ and R₃, which may be identical or different, denote a linear or branched C₁-C₃₀ alkyl or alkenyl radical or an aryl radical, at least one of the carbon atoms possibly being replaced with a heteroatom chosen from N, S, O and P;
A⁻ is a physiologically acceptable anionic counterion such as a halide, for instance a chloride or bromide, or a mesylate.

The groups L, L' and L" represent a group of formula: 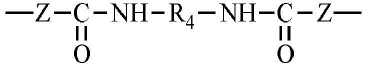 in which:
Z represents -O-, -S- or -NH-; and
R₄ represents a linear or branched alkylene radical containing from 1 to 20 carbon atoms, optionally comprising a saturated or unsaturated ring, or an arylene radical, one or more of the carbon atoms possibly being replaced with a heteroatom chosen from N, S, O and P.

The groups P and P' comprising an amine function may represent at least one of the following formulae: 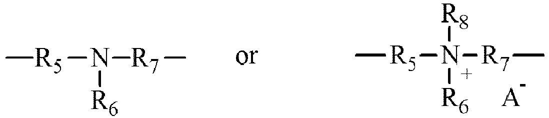 or 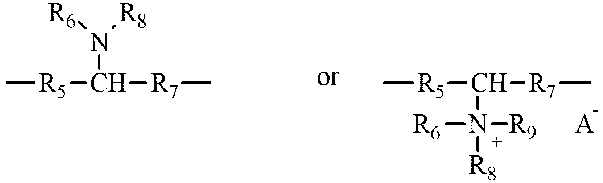 or 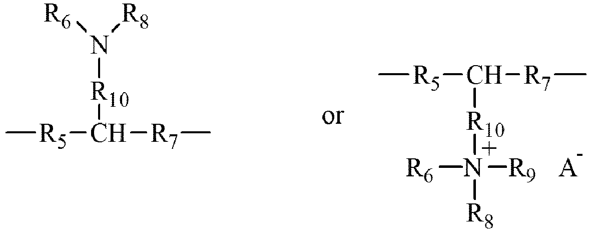 in which:
R₅ and R₇ have the same meanings as R₂ defined above;
R₆, R₈ and R₉ have the same meanings as R₁ et R₃ defined above;
R₁₀ represents a linear or branched, optionally unsaturated alkylene group possibly containing one or more heteroatoms chosen from N, O, S and P;
and A⁻ is a physiologically acceptable anionic counterion such as a halide, for instance chloride or bromide, or mesylate.

As regards the meaning of Y, the term "hydrophilic group" is intended to mean a polymeric or non-polymeric water-soluble group.

By way of example, when it is not a polymer, mention may be made of ethylene glycol, diethylene glycol and propylene glycol.

When it is a hydrophilic polymer, in accordance with one preferred embodiment, mention may be made, for example, of polyethers, sulfonated polyesters, sulfonated polyamides or a mixture of these polymers. The hydrophilic compound is preferentially a polyether and in particular a polyethylene oxide) or polypropylene oxide).

The cationic associative polyurethanes of formula (Ia) according to the invention are formed from diisocyanates and from various compounds bearing functions containing a labile hydrogen. The functions containing a labile hydrogen may be alcohol, primary or secondary amine or thiol functions, giving, after reaction with the diisocyanate functions, polyurethanes, polyureas and polythioureas, respectively. The term "polyurethanes" in the present invention encompasses these three types of polymer, namely polyurethanes per se, polyureas and polythioureas, and also copolymers thereof.

A first type of compound involved in the preparation of the polyurethane of formula (Ia) is a compound comprising at least one unit bearing an amine function. This compound may be multifunctional, but the compound is preferentially difunctional, that is to say that, according to one preferential embodiment, this compound comprises two labile hydrogen atoms borne, for example, by a hydroxyl, primary amine, secondary amine or thiol function. A mixture of multifunctional and difunctional compounds in which the percentage of multifunctional compounds is low may also be used.

As mentioned above, this compound may comprise more than one unit containing an amine function. In this case, it is a polymer bearing a repetition of the unit containing an amine function.

This type of compound may be represented by one of the following formulae: HZ-(P)ₙ-ZH, ou HZ-(P')ₚ-ZH, in which Z, P, P', n and p are as defined above.

By way of example, mention may be made of N-methyldiethanolamine, N-tert-butyldiethanolamine and N-sulfoethyldiethanolamine.

The second compound included in the preparation of the polyurethane of formula (Ia) is a diisocyanate corresponding to the formula:
O=C=N-R₄-N=C=O in which R₄ is defined above.

By way of example, mention may be made of methylenediphenyl diisocyanate, methylenecyclohexane diisocyanate, isophorone diisocyanate, tolylene diisocyanate, naphthalene diisocyanate, butane diisocyanate and hexane diisocyanate.

A third compound involved in the preparation of the polyurethane of formula (Ia) is a hydrophobic compound intended to form the terminal hydrophobic groups of the polymer of formula (Ia).

This compound is formed from a hydrophobic group and a function containing a labile hydrogen, for example a hydroxyl, primary or secondary amine, or thiol function.

By way of example, this compound may be a fatty alcohol such as stearyl alcohol, dodecyl alcohol or decyl alcohol. When this compound comprises a polymeric chain, it may be, for example, α-hydroxylated hydrogenated polybutadiene.

The hydrophobic group of the polyurethane of formula (Ia) may also result from the quaternization reaction of the tertiary amine of the compound comprising at least one tertiary amine unit. Thus, the hydrophobic group is introduced via the quaternizing agent. This quaternizing agent is a compound of the type RQ or R'Q, in which R and R' are as defined above and Q denotes a leaving group such as a halide, a sulfate, etc.

The cationic associative polyurethane may also comprise a hydrophilic block. This block is provided by a fourth type of compound involved in the preparation of the polymer. This compound may be multifunctional. It is preferably difunctional. It is also possible to have a mixture in which the percentage of multifunctional compound is low.

The functions containing a labile hydrogen are alcohol, primary or secondary amine, or thiol functions. This compound may be a polymer terminated at the chain ends with one of these functions containing a labile hydrogen.

By way of example, when it is not a polymer, mention may be made of ethylene glycol, diethylene glycol and propylene glycol.

When it is a hydrophilic polymer, mention may be made, by way of example, of polyethers, sulfonated polyesters and sulfonated polyamides, or a mixture of these polymers. The hydrophilic compound is a polyether and in particular a polyethylene oxide) or polypropylene oxide).

The hydrophilic group termed Y in formula (Ia) is optional. Specifically, the units containing a quaternary amine or protonated function may suffice to provide the solubility or water-dispersibility required for this type of polymer in an aqueous solution.

Although the presence of a hydrophilic group Y is optional, cationic associative polyurethanes comprising such a group are, however, preferred.
- (B) quaternized cellulose derivatives, and in particular:
- i) quaternized celluloses modified with groups comprising at least one fatty chain, such as linear or branched alkyl, linear or branched arylalkyl or linear or branched alkylaryl groups comprising at least 8 carbon atoms, or mixtures thereof;
- ii) quaternized hydroxyethylcelluloses modified with groups comprising at least one fatty chain, such as linear or branched alkyl, linear or branched arylalkyl or linear or branched alkylaryl groups comprising at least 8 carbon atoms, or mixtures thereof;
- iii) the hydroxyethylcelluloses of formula (Ib): 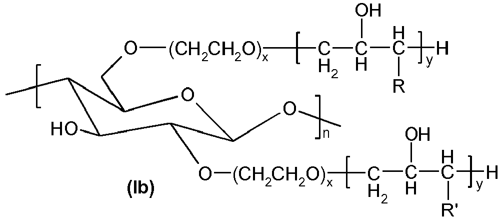 in which:
   R and R', which may be identical or different, represent an ammonium group RaR-bRcN⁺-, Q⁻ in which Ra, Rb and Rc, which may be identical or different, represent a hydrogen atom or a linear or branched C₁-C₃₀ and preferentially C₁-C₂₀ alkyl, such as methyl or dodecyl group; and Q⁻ represents an anionic counterion such as a halide, for instance a chloride or bromide; and
   n, x and y, which may be identical or different, represent an integer between 1 and 10 000.

The alkyl radicals borne by the above quaternized celluloses i) or hydroxyethylcelluloses ii) preferably comprise from 8 to 30 carbon atoms. The aryl radicals preferably denote phenyl, benzyl, naphthyl or anthryl groups.

As examples of alkylhydroxyethylcelluloses that are quaternized with C₈-C₃₀ fatty chains, mention may be made of the product Quatrisoft LM 200^{®}, sold by the company Amerchol/Dow Chemical (INCI name Polyquaternium-24) and the products Crodacel QM^{®} (INCI name PG-Hydroxyethylcellulose cocodimonium chloride), Crodacel QL^{®} (C₁₂ alkyl) (INCl name PG-Hydroxyethylcellulose lauryldimonium chloride) and Crodacel QS^{®} (C₁₈ alkyl) (INCI name PG-Hydroxyethylcellulose stearyldimonium chloride) sold by the company Croda. Mention may also be made of the hydroxyethylcelluloses of formula (lb) in which R represents a trimethylammonium halide and R' represents a dimethyldodecylammonium halide, more preferentially R represents trimethylammonium chloride (CH₃)3N⁺ Cl⁻ and R' represents dimethyldodecylammonium chloride (CH₃)₂(C₁₂H₂₅)N⁺ Cl⁻. This type of polymer is known under the INCI name Polyquaterniun-67; as commercial products, mention may be made of the Softcat Polymer SL^{®} polymers, such as SL-100, SL-60, SL-30 and SL-5, from the company Amerchol/Dow Chemical.

More particularly, the polymers of formula (Ib) are those whose viscosity is between 2000 and 3000 cPs inclusive. Preferentially, the viscosity is between 2700 and 2800 cPs inclusive. Typically, Softcat Polymer SL-5 has a viscosity of 2500 cPs, Softcat Polymer SL-30 has a viscosity of 2700 cPs, Softcat Polymer SL-60 has a viscosity of 2700 cPs and Softcat Polymer SL-100 has a viscosity of 2800 cPs.
- (C) cationic polyvinyllactams, in particular those comprising units derived (obtained by polymerisation) from:
   - a) at least one monomer of vinyllactam or alkylvinyllactam type;
   - b) at least one monomer of structure (Ic) or (IIc) below: 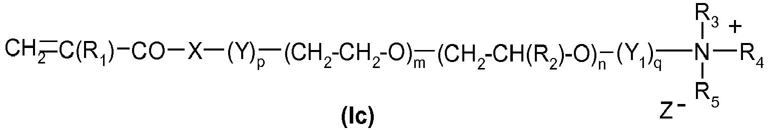 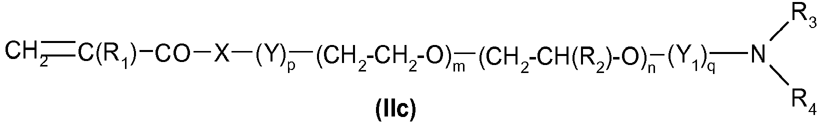 in which:
      - X denotes an oxygen atom or an NR₆ radical,
      - R₁ and R₂ denote, independently of one another, a hydrogen atom or a linear or branched C₁-C₅ alkyl radical,
      - R₂ denotes a linear or branched C₁-C₄ alkyl radical,
      - R₃, R₄ and R₅ denote, independently of one another, a hydrogen atom, a linear or branched C₁-C₃₀ alkyl radical or a radical of formula (IIIc):

         -(Y₂)ᵣ-(CH₂-CH(R₇)-O)ₓ-R₈ **(IIIc)**
      - Y, Y₁ and Y₂ denote, independently of one another, a linear or branched C₂-C₁₆ alkylene radical,
      - R₇ denotes a hydrogen atom, a linear or branched C₁-C₄ alkyl radical or a linear or branched C₁-C₄ hydroxyalkyl radical,
      - R₈ denotes a hydrogen atom or a linear or branched C₁-C₃₀ alkyl radical,
      - p, q and r denote, independently of one another, 0 or 1,
      - m and n denote, independently of one another, an integer ranging from 0 to 100 inclusive,
      - x denotes an integer ranging from 1 to 100 inclusive,
      - Z denotes an anionic counterion of an organic or mineral acid, such as a halide, for instance chloride or bromide, or mesylate;
with the proviso that:
- at least one of the substituents R₃, R₄, R₅ or R₈ denotes a linear or branched C₉-C₃₀ alkyl radical,
- if m or n is other than zero, then q is equal to 1,
- if m or n is equal to zero, then p or q is equal to 0.

The cationic poly(vinyllactam) polymers according to the invention may be crosslinked or noncrosslinked and may also be block polymers.

Preferably, the counterion Z⁻ of the monomers of formula (Ic) is chosen from halide ions, phosphate ions, the methosulfate ion and the tosylate ion.

Preferably, R₃, R₄ and R₅ denote, independently of one another, a hydrogen atom or a linear or branched C₁-C₃₀ alkyl radical.

More preferentially, the monomer b) is a monomer of formula (Ic) for which, preferentially, m and n are equal to zero.

The vinyllactam or alkylvinyllactam monomer is preferably a compound of structure (IVc): 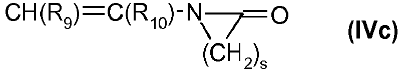 in which:
- s denotes an integer ranging from 3 to 6,
- R₉ denotes a hydrogen atom or a linear or branched C₁-C₅ alkyl radical,
- R₁₀ denotes a hydrogen atom or a linear or branched C₁-C₅ alkyl radical,
with the proviso that at least one of the radicals R₉ and R₁₀ denotes a hydrogen atom.

Even more preferentially, the monomer (IVc) is vinylpyrrolidone.

The cationic poly(vinyllactam) polymers according to the invention may also comprise units derived from one or more additional monomers, preferably cationic or non-ionic monomers.

As compounds that are particularly preferred, mention may be made of the following terpolymers comprising units derived from at least:
a)-one monomer of formula (IVc),
b)-one monomer of formula (Ic) in which p=1, q=0, R₃ and R₄ denote, independently of one another, a hydrogen atom or a C₁-C₅ alkyl radical and R₅ denotes a linear or branched C₉-C₂₄ alkyl radical, and
c)-one monomer of formula (IIc) in which R₃ and R₄ denote, independently of one another, a hydrogen atom or a linear or branched C₁-C₅ alkyl radical.

Even more preferentially, terpolymers comprising units derived from, by weight, 40% to 95% of monomer (a), 0.1% to 55% of monomer (c) and 0.25% to 50% of monomer (b) will be used. Such polymers are described, in particular, in patent application WO-00/68282.

As cationic poly(vinyllactam) polymers according to the invention, use is in particular made of:
- vinylpyrrolidone / dimethylaminopropylmethacrylamide / dodecyldimethylmethacrylamidopropylammonium tosylate terpolymers,
- vinylpyrrolidone / dimethylaminopropylmethacrylamide / cocoyldimethylmethacrylamidopropylammonium tosylate terpolymers,
- vinylpyrrolidone / dimethylaminopropylmethacrylamide / lauryldimethylmethacrylamidopropylammonium tosylate or chloride terpolymers.

The vinylpyrrolidone/dimethylaminopropylmethacrylamide/lauryldimethylmethylacrylamidopropyla mmonium chloride terpolymer is in particular provided by the company ISP under the names Styleze W10^{®} or Styleze W20L^{®} (INCI name Polyquaterniun-55).

The weight-average molecular weight (Mw) of the cationic poly(vinyllactam) polymers is preferably between 500 and 20 000 000, more particularly between 200 000 and 2 000 000 and preferentially between 400 000 and 800 000.
- (D) the cationic polymers obtained by polymerization of a monomer mixture comprising one or more vinyl monomers substituted with one or more amino groups, one or more hydrophobic non-ionic vinyl monomers, and one or more associative vinyl monomers, as described in patent application WO 2004/024779.

Among these polymers, mention will more particularly be made of the products from the polymerization of a monomer mixture comprising:
- a di(C₁-C₄ alkyl)amino(C₁-C₆ alkyl) methacrylate,
- one or more C₁-C₃₀ alkyl esters of (meth)acrylic acid,
- a polyethoxylated C₁₀-C₃₀ alkyl methacrylate (20-25 mol of ethylene oxide unit),
- a 30/5 polyethylene glycol/polypropylene glycol allyl ether,
- a hydroxy(C₂-C₆ alkyl) methacrylate, and
- an ethylene glycol dimethacrylate;

Such a polymer is for example the compound sold by the company Lubrizol under the name Carbopol Aqua CC^{®} and which corresponds to the INCI name Polyacrylate-1 crosspolymer.

Preferably, it will be possible to choose cationic polymer(s) from the associative cationic polymers, preferentially chosen from the cationic polymers (B) derived from quaternized cellulose, more particularly from the hydroxyethylcelluloses of formula (Ib) and even better still polyquaterniun-67.

The composition according to the invention may comprise the cationic polymers in an amount of between 0.01% and 2% by weight, in particular from 0.025% to 1% by weight, preferentially from 0.05% to 0.5% by weight and even better still from 0.06% to 0.2% by weight, relative to the total weight of the composition.

In particular, the composition according to the invention may comprise the associative cationic polymers in an amount of between 0.01% and 2% by weight, in particular from 0.025% to 1% by weight, preferentially from 0.05% to 0.5% by weight and even better still from 0.06% to 0.2% by weight, relative to the total weight of the composition.

### Non-ionic surfactants

The composition according to the invention may also comprise one or more non-ionic surfactants.

The non-ionic surfactants may be chosen from alcohols, α-diols and (C₁-₂₀)alkylphenols, these compounds being polyethoxylated and/or polypropoxylated and/or polyglycerolated, the number of ethylene oxide and/or propylene oxide groups possibly ranging from 1 to 100, and the number of glycerol groups possibly ranging from 2 to 30; or else these compounds comprising at least one fatty chain comprising from 8 to 30 carbon atoms and in particular from 16 to 30 carbon atoms.

Mention may also be made of condensates of ethylene oxide and of propylene oxide with fatty alcohols; polyethoxylated fatty amides preferably having from 2 to 30 ethylene oxide units, polyglycerolated fatty amides comprising on average from 1 to 5, and in particular from 1.5 to 4, glycerol groups; ethoxylated fatty acid esters of sorbitan preferably containing from 2 to 40 ethylene oxide units, fatty acid esters of sucrose, polyoxyalkylenated and preferably polyoxyethylenated fatty acid esters containing from 2 to 150 mol of ethylene oxide, including oxyethylenated plant oils, N-(C₆-₂₄ alkyl)glucamine derivatives, amine oxides such as (C₁₀-₁₄ alkyl)amine oxides or N-(C₁₀-₁₄ acyl)aminopropylmorpholine oxides.

Mention may also be made of non-ionic surfactants of alkyl(poly)glycoside type, represented in particular by general formula (VI) below:

R₂₀O-(R₂₁O)ₜ₁-(G₁)ᵥ₁

in which:
R₂₀ represents a linear or branched alkyl or alkenyl radical comprising 6 to 24 carbon atoms and in particular 8 to 18 carbon atoms, or an alkylphenyl radical whose linear or branched alkyl radical comprises 6 to 24 carbon atoms and in particular 8 to 18 carbon atoms;
R₂₁ represents an alkylene radical comprising 2 to 4 carbon atoms,
G₁ represents a sugar unit comprising 5 to 6 carbon atoms,
t₁ denotes a value ranging from 0 to 10 and preferably 0 to 4,
v₁ denotes a value ranging from 1 to 15 and preferably 1 to 4.

Preferably, the alkyl(poly)glycoside surfactants are compounds of the formula described above in which:
R₂₀ denotes a linear or branched, saturated or unsaturated alkyl radical comprising from 8 to 18 carbon atoms,
R₂₁ represents an alkylene radical comprising 2 to 4 carbon atoms,
t₁ denotes a value ranging from 0 to 3, preferably equal to 0,
G₁ denotes glucose, fructose or galactose, preferably glucose;
the degree of polymerization, i.e. the value of v₁, possibly ranging from 1 to 15 and preferably from 1 to 4; the mean degree of polymerization more particularly being between 1 and 2.

The glucoside bonds between the sugar units are generally of 1-6 or 1-4 type and preferably of 1-4 type. Preferably, the alkyl(poly)glycoside surfactant is an alkyl(poly)glucoside surfactant. C₈/C₁₆ alkyl(poly)glucosides 1,4, and in particular decyl glucosides and caprylyl/capryl glucosides, are most particularly preferred.

Among the commercial products, mention may be made of the products sold by the company Cognis under the names Plantaren^{®} (600 CS/U, 1200 and 2000) or Plantacare^{®} (818, 1200 and 2000); the products sold by the company SEPPIC under the names Oramix CG 110 and Oramix^{®} NS 10; the products sold by the company BASF under the name Lutensol GD 70, or else the products sold by the company Chem Y under the name AG10 LK.

Preferably, use is made of C₈/C₁₆-alkyl (poly)glycosides 1,4, in particular as an aqueous 53% solution, such as those sold by Cognis under the reference Plantacare^{®} 818 UP.

Preferentially, the non-ionic surfactants are chosen from (C₆-₂₄ alkyl)(poly)glycosides, and more particularly (C₈-₁₈ alkyl)(poly)glycosides, ethoxylated C₈-C₃₀ fatty acid esters of sorbitan, polyethoxylated C₈-C₃₀ fatty alcohols and polyoxyethylenated C₈-C₃₀ fatty acid esters, these compounds preferably containing from 2 to 150 mol of ethylene oxide, and mixtures thereof.

Preferably, when they are present, the composition according to the invention comprises said non-ionic surfactant(s) in an amount ranging from 0.01% to 20% by weight, in particular ranging from 0.1% to 15% by weight, preferentially from 0.2% to 10% by weight, in particular between 0.5% and 5% by weight, relative to the total weight of the composition.

### Other ingredients

Preferably, the composition according to the invention is aqueous and comprises water in a content preferably ranging from 40% to 95% by weight, in particular from 50% to 90% by weight and better still from 60% to 85% by weight, relative to the total weight of the composition.

The composition may also comprise one or more organic solvents that are liquid at 25°C and 1 atm., which are in particular water-soluble, such as C₁-C₇ alcohols, and in particular C₁-C₇ aliphatic or aromatic monoalcohols. Advantageously, the organic solvent may be chosen from ethanol and isopropanol, and mixtures thereof.

Advantageously, the composition according to the invention may also comprise one or more polyols comprising from 1 to 7 carbon atoms, in particular from 2 to 6 carbon atoms, and preferably comprising 2 or 3 hydroxyl groups; mention may be made in particular of glycerol, propylene glycol, dipropylene glycol and hexylene glycol, and also mixtures thereof.

When it comprises such polyol(s), the composition comprises it (them) preferably in an amount of between 0.05% and 10% by weight, in particular between 0.1% and 5% by weight.

The pH of the composition, if it is aqueous, is preferably inclusively between 4.5 and 9, in particular between 5 and 9, even better still between 5.5 and 8.5, in particular between 6 and 8, and preferentially between 6.5 and 7.5.

The composition according to the invention may also comprise one or more additives chosen from anionic and non-ionic polymers, ceramides, pseudo-ceramides, vitamins and provitamins including panthenol, water-soluble and liposoluble sunscreens, nacreous agents and opacifiers, thickeners, sequestrants, solubilizers, applied under the effect of heat, and may optionally be combined with chemical and/or mechanical hair treatments.

Preferably, the composition according to the invention is used as a shampoo for washing and/or conditioning the hair, or a haircare product, optionally requiring a rinsing step. Preferentially, the composition according to the invention is used as a shampoo for washing and/or conditioning the hair, followed by a rinsing step.

The compositions according to the invention may thus advantageously be in the form of a hair composition, in particular in the form of a washing hair composition, such as a shampoo, in particular a conditioning shampoo.

The present invention also relates to a cosmetic process for treating, and more particularly for washing and/or conditioning, keratin materials, in particular keratin fibres, in particular the hair, which comprises the application to said keratin materials of a composition as described above, optionally followed by a leave-on time and/or a rinsing step and/or a drying step.

The application may be performed on dry or wet hair.

The leave-on time of the composition on the keratin materials may be from 5 seconds to 10 minutes, better still from 10 seconds to 5 minutes and even better still from 20 seconds to 2 minutes.

The invention is illustrated in greater detail in the examples that follow, in which, unless otherwise mentioned, the amounts indicated are expressed as % by weight of active material (AM) of product relative to the total weight of the composition.

### Example 1

The following composition according to the invention is prepared (amounts expressed as % by weight of active material AM)

| | A1 (invention) | A2 (invention) | A3 (Reference) |
|---|---|---|---|
| Sodium lauryl ether sulfate | 11.76 | 11.76 | 11.76 |
| Sodium lauryl sulfate | 3.13 | - | - |
| Laureth-5 carboxylic acid | 2.7 | 2.7 | 2.7 |
| Cocamidopropyl betaine | 4.56 | - | - |
| (3-Am inopropyl)triethoxysilane | 0.5 | 1 | 1 |
| Lactic acid | 0.16 | 0.22 | 0.22 |
| PEG-40/PPG-8 methylaminopropyl/hydroxypropyl dimethicone copolymer (Silsoft A+) | 0.15 | 0.15 | - |
| Quaternium-80 (AbilQuat 3272) | - | - | 0.15 |
| Polyquaternium-67 | 0.1 | - | - |
| PEG-60 hydrogenated castor oil | 0.5 | - | - |
| PEG-55 propylene glycol oleate | 0.24 | - | - |
| PPG-5 ceteth-20 | 0.5 | - | - |
| pH agent | qs pH 7 | qs pH 7 | qs pH 7 |
| Preservatives, fragrances | qs | qs | qs |
| Water | qs 100% | qs 100% | qs 100% |

The compositions are all clear and stable. The turbidity of the composition A1 measured using a 2100P turbidimeter from the company Hach, at ambient temperature (25°C), is 10 NTU.

The compositions were used as a shampoo on fine hair. They all exhibit good foam initiation and generate an abundant and long-lasting foam.

After rinsing and drying, the hair is light, soft and easy to style and it is observed that the head of hair has been given body and volume.

### Example 2

The compositions below were prepared (amounts expressed in g % of active material)

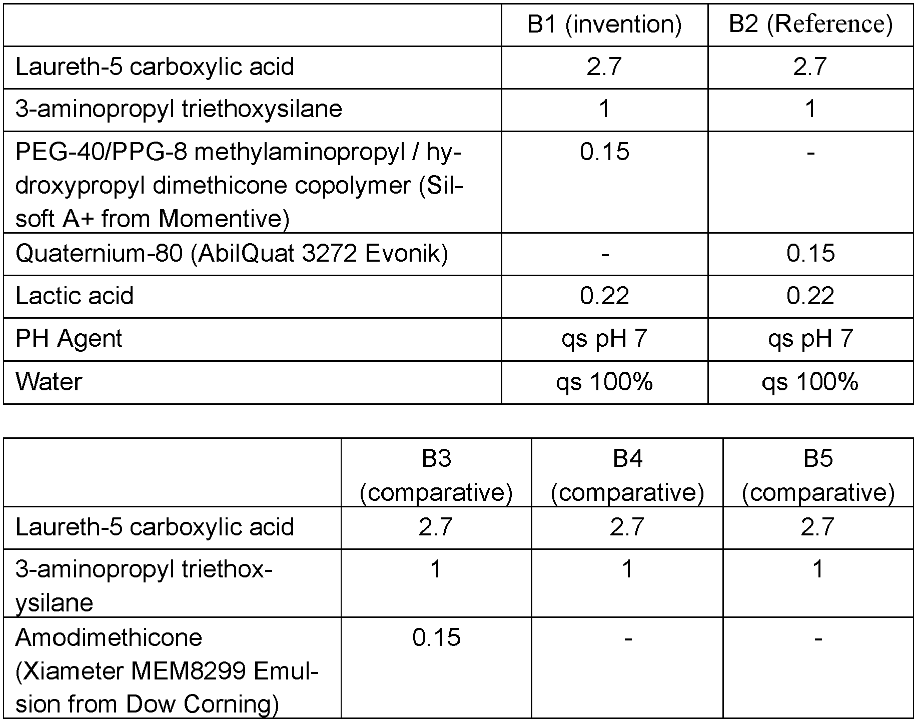

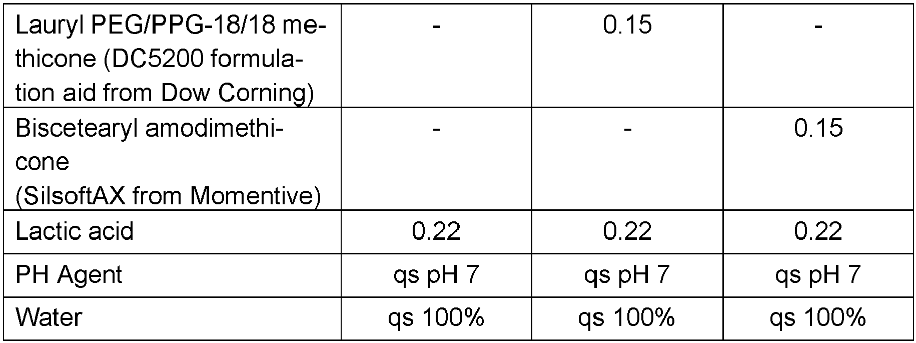

24 hours after they have been prepared, the compositions according to the invention B1 and B2 are translucent and stable, whereas the comparative compositions B3, B4 and B5 are cloudy and non-homogeneous and have flocculated.

## Claims

1. Composition, comprising:
- at least one anionic surfactant chosen from polyoxyalkylenated (C₆-C₂₄)alkyl(amido) ether carboxylic acids and salts comprising from 2 to 50 ethylene oxide groups;
- at least one organosilane chosen from the compounds of formula (I) and/or oligomers thereof:
R₁Si(OR₂)_{z}(R₃)ₓ(OH)_{y} (I)
in which:
- R₁ is a linear or branched, saturated C₁-C₂₂ hydrocarbon-based chain, which may be substituted with an amine group NH₂ or NHR (R ₌ C₁-C₂₀ alkyl),
- R₂ represents an alkyl group comprising from 1 to 4 carbon atoms,
- R₃ represents an alkyl group comprising from 1 to 4 carbon atoms,
- y is 0,
- z denotes an integer ranging from 1 to 3,
- x denotes an integer ranging from 0 to 2,
- with z + x + y = 3; and
- at least one amino silicone comprising at least one polyoxyalkylenated group, the amino silicone(s) being present in an amount ranging from 0.01% to 5% by weight, relative to the total weight of the composition.

2. Composition according to Claim 1, comprising at least one anionic surfactant chosen from polyoxyalkylenated alkyl(amido) ether carboxylic acids of formula (1):
R_{1'}-(OC₂H₄)_{n'}-OCH₂COOA (1)
in which:
- R_{1'} represents a linear or branched C₆-C₂₄ alkyl or alkenyl radical, a (C₈-C₉)alkylphenyl radical, an R_{2'}CONH-CH₂-CH₂- radical with R_{2'} denoting a linear or branched C₉-C₂₁ alkyl or alkenyl radical; preferably R_{1'} is a C₈-C₂₀, preferably C₈-C₁₈, alkyl radical and aryl preferably denotes phenyl,
- n' is an integer or decimal number (average value) ranging from 2 to 24 and preferably from 2 to 10,
- A denotes H, ammonium, Na, K, Li, Mg or a monoethanolamine or triethanolamine residue;
preferentially the polyoxyalkylenated alkyl(amido) ether carboxylic acids of formula (1) in which:
- R_{1'} denotes a linear or branched C₈-C₂₂, in particular C₁₀-C₁₆ or even C₁₂-C₁₄ alkyl radical, or alternatively a (C₈-C₉)alkylphenyl radical;
- A denotes a hydrogen or sodium atom, and
- n' ranges from 2 to 20, preferably from 2 to 10;
more preferentially, the polyoxyalkylenated alkyl(amido) ether carboxylic acids of formula (1) in which R₁' denotes a C₁₂ alkyl radical, A denotes a hydrogen or sodium atom and n' ranges from 2 to 10.

3. Composition according to any one of the preceding claims, in which the total content of anionic surfactants ranges from 1% to 35% by weight, in particular from 3% to 25% by weight, better still from 5% to 22% by weight, relative to the total weight of the composition.

4. Composition according to any one of the preceding claims, in which the organosilane is chosen from the compounds of formula (I) and/or oligomers thereof:
R₁Si(OR₂)_{z}(R₃)ₓ(OH)_{y} (I)
in which:
- R₁ is a linear, saturated C₂-C₁₂, hydrocarbon-based chain, which may be substituted with an amine group NH₂ or NHR (R = C₁-C₆, alkyl); and
- R₂ represents a linear alkyl group comprising from 1 to 4 carbon atoms, and preferably the methyl or ethyl group; and
- R₃ represents a linear alkyl group comprising from 1 to 4 carbon atoms, and preferably the methyl or ethyl group; and
- z ₌ 3 and thus x = y ₌ 0, or else z ₌ 2, x ₌ 1 and y = 0;
preferentially, R₁ represents a C₁-C₆, preferably C₂-C₄, amino alcohol group, z ₌ 3 and x ₌ y ₌ 0.

5. Composition according to any one of the preceding claims, in which the organosilane is chosen from octyltriethoxysilane (OTES), dodecyltriethoxysilane, octadecyltriethoxysilane, hexadecyltriethoxysilane, 3-aminopropyltriethoxysilane (APTES), methyltriethoxysilane (MTES), 2-aminoethyltriethoxysilane (AETES), 3-aminopropylmethyldiethoxysilane, N-(2-aminoethyl)-3-aminopropyltriethoxysilane, 3-(m-aminophenoxy)propyltrimethoxysilane, p-aminophenyltrimethoxysilane, N-(2-aminoethylaminomethyl)phenethyltrimethoxysilane, and oligomers thereof; and more particularly chosen from 3-aminopropyltriethoxysilane (APTES) and oligomers thereof.

6. Composition according to any one of the preceding claims, comprising the organosilane(s) in an amount ranging from 0.01% to 10% by weight, preferably from 0.1% to 5% by weight, even better still from 0.2% to 1% by weight, relative to the total weight of the composition.

7. Composition according to any one of the preceding claims, in which the amino silicone is chosen from polyoxyalkylenated amino silicones, of (AB)n multiblock type, A being a polysiloxane block and B being a polyoxyalkylene block, and comprising at least one amine group; in particular, the silicones constituted of repeating units of formula (111.1) and/or of formula (III.2) below:
[-(SiMe₂O)ᵥ-SiMe₂-R₇-N(R₈)-R₉-O(C₃H₆O)ₐ(C₂H₄O)_{b}-R₉-N(R₈)-R₇-] (III.1)
[-(SiMe₂O)ᵥ-SiMe₂-R₇-N(R₈)-R₉-O(C₃H₆O)ₐ(C₂H₄O)_{b}-] (III.2)
in which:
- a is an integer greater than or equal to 1, preferably ranging from 5 to 200, or even from 60 to 100, and more particularly ranging from 10 to 100;
- b is an integer between 0 and 200, preferably ranging from 4 to 100, or even from 5 to 50, and more particularly between 5 and 30;
- v is an integer ranging from 1 to 10 000 and more particularly from 10 to 5000;
- R₈, which may be identical or different, are a hydrogen atom or a methyl, preferably methyl;
- R₇, which may be identical or different, represent a linear or branched divalent C₂-C₁₂ hydrocarbon-based radical, optionally comprising one or more heteroatoms such as oxygen; preferably, R₇ denotes an ethylene radical, a linear or branched propylene radical, a linear or branched butylene radical or a CH₂CH₂CH₂OCH₂CH(OH)CH₂- radical; preferentially, R₇ represents a CH₂CH₂CH₂OCH₂CH(OH)CH₂- radical;
- R₉, which may be identical or different, represent a linear or branched C₂-C₁₂ divalent hydrocarbon-based radical, optionally comprising one or more heteroatoms such as oxygen; preferably, R₉ denotes an ethylene radical, a linear or branched propylene radical, a linear or branched butylene radical, or a CH₂CH₂CH₂OCH₂CH(OH)CH₂- radical; preferentially, R₉ denotes -CH(CH₃)-CH₂-;
preferably, the siloxane blocks -(SiMe₂O)ᵥ- representing 50 mol% to 95 mol% of the total weight of the silicone, more particularly 70 mol% to 85 mol%.

8. Composition according to any one of the preceding claims, comprising the amino silicone(s) in an amount ranging from 0.05% to 2% by weight and preferentially from 0.1% to 0.5% by weight, relative to the total weight of the composition.

9. Composition according to any one of the preceding claims, comprising one or more amphoteric surfactants, preferably chosen from (C₈-C₂₀)alkylbetaines, (C₈-C₂₀)alkylamido(C₁-C₆)alkylbetaines, (C₈-C₂₀)alkyl amphoacetates and (C₈-C₂₀)alkylamphodiacetates, and mixtures thereof, and in particular from cocobetaine and cocoamidopropylbetaine, preferably in an amount ranging from 1% to 15% by weight, in particular ranging from 2% to 10% by weight, preferentially from 3% to 8% by weight, relative to the total weight of the composition.

10. Composition according to any one of the preceding claims, comprising one or more cationic polymers; preferably chosen from associative cationic polymers; and even better still chosen from:
- (A) the cationic associative polyurethanes of general formula (Ia) below: R-X-(P)n-[L-(Y)m]r-L'-(P')p-X'-R'
in which:
R and R', which may be identical or different, represent a hydrophobic group or a hydrogen atom;
X and X', which may be identical or different, represent a group comprising an amine function optionally bearing a hydrophobic group, or alternatively the group L";
L, L' and L", which may be identical or different, represent a group derived from a diisocyanate;
P and P', which may be identical or different, represent a group comprising an amine function optionally bearing a hydrophobic group;
Y represents a hydrophilic group;
r is an integer between 1 and 100 inclusive, preferably between 1 and 50 inclusive and in particular between 1 and 25 inclusive;
n, m and p are each, independently of one another, between 0 and 1000 inclusive;
the molecule containing at least one protonated or quaternized amine function and at least one hydrophobic group;
- (B) quaternized cellulose derivatives, and in particular:
- i) quaternized celluloses modified with groups comprising at least one fatty chain, such as linear or branched alkyl, linear or branched arylalkyl or linear or branched alkylaryl groups comprising at least 8 carbon atoms, or mixtures thereof;
- ii) quaternized hydroxyethylcelluloses modified with groups comprising at least one fatty chain, such as linear or branched alkyl, linear or branched arylalkyl or linear or branched alkylaryl groups comprising at least 8 carbon atoms, or mixtures thereof;
- iii) the hydroxyethylcelluloses of formula (Ib): 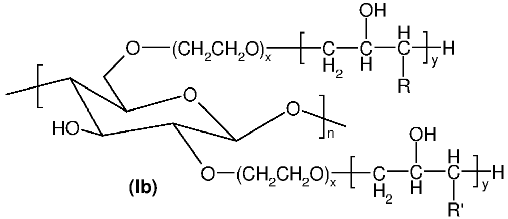 in which:
R and R', which may be identical or different, represent an ammonium group RaR-bRcN⁺-, Q⁻ in which Ra, Rb and Rc, which may be identical or different, represent a hydrogen atom or a linear or branched C₁-C₃₀ and preferentially C₁-C₂₀ alkyl, such as methyl or dodecyl group; and Q⁻ represents an anionic counterion such as a halide, for instance a chloride or bromide; and
n, x and y, which may be identical or different, represent an integer between 1 and 10 000, the alkyl radicals borne by the celluloses i) or quaternised hydroxyethylcelluloses ii) above preferably comprise from 8 to 30 carbon atoms, the aryl radicals preferably denote phenyl, benzyl, naphthyl or anthryl groups;
- (C) cationic polyvinyllactams, in particular those comprising units derived from:
- a) at least one monomer of vinyllactam or alkylvinyllactam type;
- b) at least one monomer of structure (Ic) or (IIc) below: 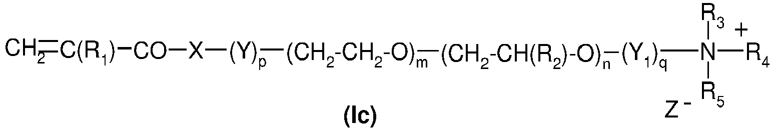 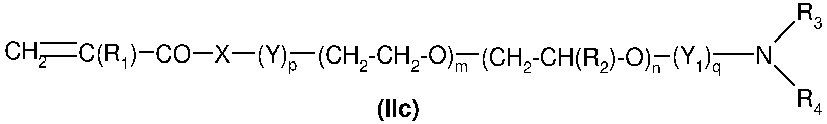 in which:
- X denotes an oxygen atom or an NR₆ radical,
- R₁ and R₆ denote, independently of one another, a hydrogen atom or a linear or branched C₁-C₅ alkyl radical,
- R₂ denotes a linear or branched C₁-C₄ alkyl radical,
- R₃, R₄ and R₅ denote, independently of one another, a hydrogen atom, a linear or branched C₁-C₃₀ alkyl radical or a radical of formula (IIIc):
-(Y₂)ᵣ-(CH₂-CH(R₇)-O)ₓ-R₈ **(IIIc)**
- Y, Y₁ and Y₂ denote, independently of one another, a linear or branched C₂-C₁₆ alkylene radical,
- R₇ denotes a hydrogen atom, or a linear or branched C₁-C₄ alkyl radical or a linear or branched C₁-C₄ hydroxyalkyl radical,
- R₈ denotes a hydrogen atom or a linear or branched C₁-C₃₀ alkyl radical,
- p, q and r denote, independently of one another, either the value zero or the value 1,
- m and n denote, independently of one another, an integer ranging from 0 to 100 inclusive,
- x denotes an integer ranging from 1 to 100 inclusive,
- Z denotes an anionic counterion of an organic or mineral acid, such as a halide, for instance chloride or bromide, or mesylate;
with the proviso that:
- at least one of the substituents R₃, R₄, R₅ or R₈ denotes a linear or branched C₉-C₃₀ alkyl radical,
- if m or n is other than zero, then q is equal to 1,
- if m or n is equal to zero, then p or q is equal to 0.
- (D) the cationic polymers obtained by polymerization of a monomer mixture comprising one or more vinyl monomers substituted with one or more amino groups, one or more hydrophobic non-ionic vinyl monomers, and one or more associative vinyl monomers.

11. Composition according to any one of the preceding claims, comprising one or more cationic polymers (B) derived from quaternised cellulose, particularly chosen from the hydroxyethylcelluloses of formula (Ib) and even better still polyquaternium-67.

12. Composition according to any one of the preceding claims, comprising the cationic polymers in an amount of between 0.01% and 2% by weight, in particular from 0.025% to 1% by weight, preferentially from 0.05% to 0.5% by weight and even better still from 0.06% to 0.2% by weight, relative to the total weight of the composition; and/or comprising the associative cationic polymers in an amount of between 0.01% and 2% by weight, in particular from 0.025% to 1% by weight, preferentially from 0.05% to 0.5% by weight and even better still from 0.06% to 0.2% by weight, relative to the total weight of the composition.

13. Composition according to any one of the preceding claims, comprising one or more non-ionic surfactants, preferably chosen from:
- alcohols, α-diols and (C₁-₂₀)alkylphenols, these compounds being polyethoxylated and/or polypropoxylated and/or polyglycerolated, the number of ethylene oxide and/or propylene oxide groups possibly ranging from 1 to 100, and the number of glycerol groups possibly ranging from 2 to 30; or else these compounds comprising at least one fatty chain comprising from 8 to 30 carbon atoms and in particular from 16 to 30 carbon atoms;
- condensates of ethylene oxide and of propylene oxide with fatty alcohols; polyethoxylated fatty amides preferably having from 2 to 30 ethylene oxide units, polyglycerolated fatty amides comprising on average from 1 to 5, and in particular from 1.5 to 4, glycerol groups; ethoxylated fatty acid esters of sorbitan preferably containing from 2 to 40 ethylene oxide units, fatty acid esters of sucrose, polyoxyalkylenated and preferably polyoxyethylenated fatty acid esters containing from 2 to 150 mol of ethylene oxide, including oxyethylenated plant oils, N-(C₆-₂₄ alkyl)glucamine derivatives, amine oxides such as (C₁₀-₁₄ alkyl)amine oxides or N-(C₁₀-₁₄ acyl)aminopropylmorpholine oxides;
- non-ionic surfactants of alkyl(poly)glycoside type, represented in particular by general formula (VI) below:
R₂₀O-(R₂₁O)ₜ₁-(G₁)ᵥ₁
in which:
R₂₀ represents a linear or branched alkyl or alkenyl radical comprising 6 to 24 carbon atoms and in particular 8 to 18 carbon atoms, or an alkylphenyl radical whose linear or branched alkyl radical comprises 6 to 24 carbon atoms and in particular 8 to 18 carbon atoms;
R₂₁ represents an alkylene radical comprising 2 to 4 carbon atoms,
G₁ represents a sugar unit comprising 5 to 6 carbon atoms,
t₁ denotes a value ranging from 0 to 10 and preferably 0 to 4,
v₁ denotes a value ranging from 1 to 15 and preferably 1 to 4;
preferably in which:
R₂₀ denotes a linear or branched, saturated or unsaturated alkyl radical comprising from 8 to 18 carbon atoms,
R₂₁ represents an alkylene radical comprising 2 to 4 carbon atoms,
t₁ denotes a value ranging from 0 to 3, preferably equal to 0,
G₁ denotes glucose, fructose or galactose, preferably glucose;
the degree of polymerization, i.e. the value of v₁, possibly ranging from 1 to 15 and preferably from 1 to 4; the mean degree of polymerization more particularly being between 1 and 2;
preferably present in an amount ranging from 0.01% to 20% by weight, preferably ranging from 0.1% to 15% by weight, better still from 0.2% to 10% by weight and preferentially from 0.5% to 5% by weight, relative to the total weight of the composition.

14. Composition according to any one of the preceding claims, comprising water in a content preferably ranging from 40% to 95% by weight, in particular from 50% to 90% by weight and better still from 60% to 85% by weight, relative to the total weight of the composition.

15. Composition according to any one of the preceding claims, having a pH inclusively between 4.5 and 9, in particular between 5 and 9, even better still between 5.5 and 8.5, in particular between 6 and 8, and preferentially between 6.5 and 7.5.

16. Cosmetic process for treating, and more particularly for washing and/or conditioning, keratin materials, in particular keratin fibres, in particular the hair, which comprises the application to said keratin materials of a composition according to any one of the preceding claims, optionally followed by a leave-on time and/or a rinsing step and/or a drying step.

## Patentansprüche

1. Zusammensetzung, umfassend:
- mindestens ein anionisches Tensid, ausgewählt aus polyoxyalkylenierten (C₆-C₂₄)Alkyl (amido) ethercarbonsäuren und Salzen davon mit 2 bis 50 Ethylenoxidgruppen;
- mindestens ein Organosilan, ausgewählt aus den Verbindungen der Formel **(I)** und/oder Oligomeren davon:
R₁Si(OR₂)_{z}(R₃)ₓ(OH)_{y} (I)
wobei:
- R₁ für eine lineare oder verzweigte, gesättigte C₁-C₂₂-Kette auf Kohlenwasserstoffbasis, die durch eine Amingruppe NH₂ oder NHR (R = C₁-C₂₀-Alkyl) substituiert sein kann, steht,
- R₂ für eine Alkylgruppe mit 1 bis 4 Kohlenstoffatomen steht,
- R₃ für eine Alkylgruppe mit 1 bis 4 Kohlenstoffatomen steht,
- y für 0 steht,
- z eine ganze Zahl im Bereich von 1 bis 3 bedeutet,
- x eine ganze Zahl im Bereich von 0 bis 2 bedeutet,
- wobei z + x + y = 3; und
- mindestens ein Aminosilikon mit mindestens einer polyoxyalkylenierten Gruppe, wobei das Aminosilikon bzw. die Aminosilikone in einer Menge im Bereich von 0,01 bis 5 Gew.-%, bezogen auf das Gesamtgewicht der Zusammensetzung, vorliegt bzw. vorliegen.

2. Zusammensetzung nach Anspruch 1, umfassend mindestens ein anionisches Tensid, ausgewählt aus polyoxyalkylenierten Alkyl(amido)ethercarbonsäuren der Formel (1):
R_{1'}-(OC₂H₄)_{n'}-OCH₂COOA (1)
wobei:
- R_{1'} für einen linearen oder verzweigten C₆-C₂₄-Alkyl- oder -Alkenylrest, einen (C₈-C₉)Alkylphenylrest oder einen R_{2'}CONH-CH₂-CH₂-Rest, wobei R_{2'} einen linearen oder verzweigten C₉-C₂₁-Alkyl- oder
- Alkenylrest bedeutet, steht; vorzugsweise R_{1'} für einen C₈-C₂₀- und vorzugsweise C₈-C₁₈-Alkylrest steht und Aryl vorzugsweise Phenyl bedeutet,
- n' für eine ganze Zahl oder Dezimalzahl (durchschnittlicher Wert) im Bereich von 2 bis 24 und vorzugsweise von 2 bis 10 steht,
- A H, Ammonium, Na, K, Li, Mg oder einen Monoethanolamin- oder Triethanolamin-Rest bedeutet; bevorzugt den polyoxyalkylenierten Alkyl(amido)-ethercarbonsäuren der Formel (1), wobei:
- R_{1'} für einen linearen oder verzweigten C₈-C₂₂-, insbesondere C₁₀-C₁₆- oder sogar C₁₂-C₁₄-Alkylrest oder auch einen (C₈-C₉)-Alkylphenylrest steht;
- A ein Wasserstoff- oder Natriumatom bedeutet und
- n' im Bereich von 2 bis 20, vorzugsweise von 2 bis 10, liegt;
weiter bevorzugt den polyoxyalkylenierten Alkyl-(amido)ethercarbonsäuren der Formel (1), wobei R_{1'} einen C₁₂-Alkylrest bedeutet, A ein Wasserstoff- oder Natriumatom bedeutet und n' im Bereich von 2 bis 10 liegt.

3. Zusammensetzung nach einem der vorhergehenden Ansprüche, wobei der Gesamtgehalt von anionischen Tensiden im Bereich von 1 bis 35 Gew.-%, insbesondere von 3 bis 25 Gew.-%, noch besser von 5 bis 22 Gew.-%, bezogen auf das Gesamtgewicht der Zusammensetzung, liegt.

4. Zusammensetzung nach einem der vorhergehenden Ansprüche, wobei das Organosilan, ausgewählt aus den Verbindungen der Formel **(I)** und/oder Oligomeren davon ausgewählt ist:
R₁Si(OR₂)_{z}(R₃)ₓ(OH)_{y} (I)
wobei:
- R₁ für eine lineare, gesättigte C₂-C₁₂-Kette auf Kohlenwasserstoffbasis, die durch eine Amingruppe NH₂ oder NHR (R = C₁-C₆-Alkyl) substituiert sein kann, steht; und
- R₂ für eine lineare Alkylgruppe mit 1 bis 4 Kohlenstoffatomen und vorzugsweise die Methyl- oder Ethylgruppe steht; und
- R₃ für eine Alkylgruppe mit 1 bis 4 Kohlenstoffatomen und vorzugsweise die Methyl- oder Ethylgruppe steht; und
- z = 3 und somit x = y = 0 oder auch z = 2, x = 1 und y = 0,
bevorzugt R₁ für eine C₁-C₆- und vorzugsweise C₂-C₄-Aminoalkoholgruppe steht, z = 3 und x = y = 0.

5. Zusammensetzung nach einem der vorhergehenden Ansprüche, wobei das Organosilan aus Octyltriethoxysilan (OTES), Dodecyltriethoxysilan, Octadecyltriethoxysilan, Hexadecyltriethoxysilan, 3-Aminopropyltriethoxysilan (APTES), Methyltriethoxysilan (MTES), 2-Aminoethyltriethoxysilan (AETES), 3-Aminopropylmethyldiethoxysilan, N-(2-Aminoethyl)-3-aminopropyltriethoxysilan, 3-(m-Amino-phenoxy)propyltrimethoxysilan, p-Aminophenyltrimethoxysilan, N-(2-Aminoethylaminomethyl)phenethyl-trimethoxysilan und Oligomeren davon ausgewählt ist und spezieller aus 3-Aminopropyltriethoxysilan (APTES) und Oligomeren davon ausgewählt ist.

6. Zusammensetzung nach einem der vorhergehenden Ansprüche, umfassend das Organosilan bzw. die Organosilane in einer Menge im Bereich von 0,01 bis 10 Gew.-%, vorzugsweise von 0,1 bis 5 Gew.-%, noch besser von 0,2 bis 1 Gew.-%, bezogen auf das Gesamtgewicht der Zusammensetzung.

7. Zusammensetzung nach einem der vorhergehenden Ansprüche, wobei das Aminosilikon aus polyoxyalkylenierten Aminosilikonen vom (AB)n-Mehrblock-Typ, wobei A für einen Polysiloxanblock steht und B für einen Polyoxyalkylenblock steht, und mit mindestens einer Amingruppe ausgewählt ist; insbesondere den Silikonen, die aus Wiederholungseinheiten der nachstehenden Formel (III.1) und/oder Formel (III.2) aufgebaut sind:
[-(SiMe₂O)ᵥ-SiMe₂-R₇-N(R₈)-R₉-O(C₃H₆O)ₐ(C₂H₄O)_{b}-R₉-N(R₈)-R₇-] (III.1)
[-(SiMe₂O)ᵥ-SiMe₂-R₇-N(R₈)-R₉-O(C₃H₆O)ₐ(C₂H₄O)_{b}-] (III.2)
wobei:
- a für eine ganze Zahl größer oder gleich 1, vorzugsweise im Bereich von 5 bis 200 oder sogar von 60 bis 100 und spezieller im Bereich von 10 bis 100 steht;
- b für eine ganze Zahl zwischen 0 und 200, vorzugsweise im Bereich von 4 bis 100 oder sogar von 5 bis 50 und spezieller zwischen 5 und 30 steht;
- v für eine ganze Zahl im Bereich von 1 bis 10.000 und spezieller von 10 bis 5000 steht;
- R₈, die gleich oder verschieden sein können, für ein Wasserstoffatom oder ein Methyl, vorzugsweise Methyl, stehen;
- R₇, die gleich oder verschieden sein können, für einen linearen oder verzweigten zweiwertigen C₂-C₁₂-Rest auf Kohlenwasserstoffbasis, der gegebenenfalls ein oder mehrere Heteroatome wie Sauerstoff umfasst, steht; vorzugsweise R₇ einen Ethylenrest, einen linearen oder verzweigten Propylenrest, einen linearen oder verzweigten Butylenrest oder einen CH₂CH₂CH₂OCH₂CH(OH)CH₂-Rest bedeutet; bevorzugt R₇ für einen CH₂CH₂CH₂OCH₂CH(OH)CH₂-Rest steht;
- R₉, die gleich oder verschieden sein können, für einen linearen oder verzweigten zweiwertigen C₂-C₁₂-Rest auf Kohlenwasserstoffbasis, der gegebenenfalls ein oder mehrere Heteroatome wie Sauerstoff umfasst, steht; vorzugsweise R₉ einen Ethylenrest, einen linearen oder verzweigten Propylenrest, einen linearen oder verzweigten Butylenrest oder einen CH₂CH₂CH₂OCH₂CH(OH)CH₂-Rest bedeutet; bevorzugt R₉ für -CH(CH₃)-CH₂- steht;
wobei vorzugsweisedie Siloxanblöcke -(SiMe₂O)ᵥ- 50 Mol-% bis 95 Mol-% des Gesamtgewichts des Silikons, spezieller 70 Mol-% bis 85 Mol-%, ausmachen.

8. Zusammensetzung nach einem der vorhergehenden Ansprüche, umfassend das Aminosilikon bzw. die Aminosilikone in einer Menge im Bereich von 0,05 bis 2 Gew.-% und bevorzugt von 0,1 bis 0,5 Gew.-%, bezogen auf das Gesamtgewicht der Zusammensetzung.

9. Zusammensetzung nach einem der vorhergehenden Ansprüche, umfassend ein oder mehrere amphotere Tenside, vorzugsweise ausgewählt aus (C₈-C₂₀)Alkylbetainen, (C₈-C₂₀) Alkylamido (C₁-C₆) alkylbetainen, (C₈-C₂₀) Alkylamphoacetaten und (C₈-C₂₀) Alkylamphodiacetaten und Mischungen davon und insbesondere aus Cocobetain und Cocoamidopropylbetain, vorzugsweise in einer Menge im Bereich von 1 bis 15 Gew.-%, insbesondere im Bereich von 2 bis 10 Gew.-%, bevorzugt von 3 bis 8 Gew.-%, bezogen auf das Gesamtgewicht der Zusammensetzung.

10. Zusammensetzung nach einem der vorhergehenden Ansprüche, umfassend ein oder mehrere kationische Polymere; vorzugsweise ausgewählt aus assoziativen kationischen Polymeren und noch besser ausgewählt aus:
- (A) den kationischen assoziativen Polyurethanen der nachstehenden allgemeinen Formel (Ia): R-X-(P)n-[L-(Y)m]r-L'-(P')ₚ-X'-R', wobei:
R und R', die gleich oder verschieden sein können, für eine hydrophobe Gruppe oder ein Wasserstoffatom stehen;
X und X', die gleich oder verschieden sein können, für eine Gruppe mit einer Aminfunktion, die gegebenenfalls eine hydrophobe Gruppe trägt, oder auch die Gruppe L" stehen;
L, L' und L", die gleich oder verschieden sein können, für eine von einem Diisocyanat abgeleitete Gruppe stehen;
P und P', die gleich oder verschieden sein können, für eine Gruppe mit einer Aminfunktion, die gegebenenfalls eine hydrophobe Gruppe trägt, stehen;
Y für eine hydrophile Gruppe steht;
r für eine ganze Zahl zwischen 1 und 100 inklusive, vorzugsweise zwischen 1 und 50 inklusive und insbesondere zwischen 1 und 25 inklusive steht;
n, m und p jeweils unabhängig voneinander Werte zwischen 0 und 1000 inklusive aufweisen;
wobei das Molekül mindestens eine protonierte oder quaternisierte Aminfunktion und mindestens eine hydrophobe Gruppe enthält;
- (B) quaternisierten Cellulosederivaten und insbesondere
- i) quaternisierten Cellulosen, die mit Gruppen mit mindestens einer Fettkette, wie linearen oder verzweigten Alkyl-, linearen oder verzweigten Arylalkyl- oder linearen oder verzweigten Alkylarylgruppen mit mindestens 8 Kohlenstoffatome oder Mischungen davon, modifiziert sind;
- ii) quaternisierten Hydroxyethylcellulosen, die mit Gruppen mit mindestens einer Fettkette, wie linearen oder verzweigten Alkyl-, linearen oder verzweigten Arylalkyl- oder linearen oder verzweigten Alkylarylgruppen mit mindestens 8 Kohlenstoffatome oder Mischungen davon, modifiziert sind;
- iii) den Hydroxyethylcellulosen der Formel (Ib): 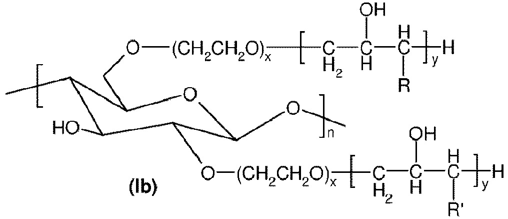 wobei:
R und R', die gleich oder verschieden sein können, für eine Ammoniumgruppe RaRbRcN⁺-, Q⁻ stehen, wobei Ra, Rb und Rc, die gleich oder verschieden sein können, für ein Wasserstoffatom oder eine lineare oder verzweigte C₁-C₃₀- und bevorzugt C₁-C₂₀-Alkylgruppe, wie eine Methyl- oder Dodecylgruppe, stehen und Q⁻ für ein anionisches Gegenion wie ein Halogenid, beispielsweise ein Chlorid oder Bromid, steht; und
n, x und y, die gleich oder verschieden sein können, für eine ganze Zahl zwischen 1 und 10.000 stehen, wobei die von den Cellulosen i) oder quaternisierten Hydroxyethylcellulosen ii) oben getragenen Alkylreste vorzugsweise 8 bis 30 Kohlenstoffatomen umfassen und die Arylreste vorzugsweise Phenyl-, Benzyl-, Naphthyl- oder Anthrylgruppen bedeuten;
- (C) kationischen Polyvinyllactamen, insbesondere denjenigen, die Einheiten umfassen, die sich von Folgendem ableiten:
- a) mindestens einem Monomer vom Vinyllactam- oder Alkylvinyllactam-Typ;
- b) mindestens einem Monomer der nachstehenden Struktur (Ic) oder (IIc): 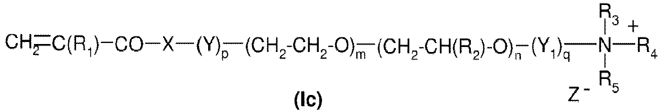 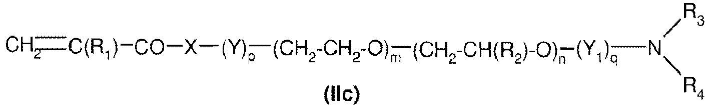 wobei:
- X ein Sauerstoffatom oder einen NR₆-Rest bedeutet,
- R₁ und R₆ unabhängig voneinander ein Wasserstoffatom oder einen linearen oder verzweigten C₁-C₅-Alkylrest bedeuten,
- R₂ einen linearen oder verzweigten C₁-C₄-Alkylrest bedeutet,
- R₃, R₄ und R₅ unabhängig voneinander ein Wasserstoffatom, einen linearen oder verzweigten C₂-C₃₀-Alkylrest oder einen Rest der Formel (IIIc):
-(Y₂)ᵣ-(CH₂-CH(R₇)-O)ₓ-R₈ **(IIIc)**
bedeuten,
- Y, Y₁ und Y₂ unabhängig voneinander einen linearen oder verzweigten C₂-C₁₆-Alkylenrest bedeuten,
- R₇ ein Wasserstoffatom oder einen linearen oder verzweigten C₂-C₄-Alkylrest oder einen linearen oder verzweigten C₁-C₄-Hydroxyalkylrest bedeutet,
- R₈ ein Wasserstoffatom oder einen linearen oder verzweigten C₁-C₃₀-Alkylrest bedeutet,
- p, q und r unabhängig voneinander entweder den Wert 0 oder den Wert 1 bedeuten,
- m und n unabhängig voneinander eine ganze Zahl im Bereich von 0 bis 100 inklusive bedeuten,
- x eine ganze Zahl im Bereich von 1 bis 100 inklusive bedeutet,
- Z ein anionisches Gegenion einer organischen Säure oder Mineralsäure, wie ein Halogenid, beispielsweise Chlorid oder Bromid, oder Mesylat, bedeutet;
mit der Maßgabe, dass:
- mindestens einer der Substituenten R₃, R₄, R₅ oder R₈ einen linearen oder verzweigten C₉-C₃₀-Alkylrest bedeutet,
- dann, wenn m oder n von null verschieden ist, q gleich 1 ist,
- dann, wenn m oder n gleich null ist, p oder q gleich 0 ist;
- (D) den kationischen Polymeren, die durch Polymerisation einer Monomerenmischung, die ein oder mehrere Vinylmonomere, die durch eine oder mehrere Aminogruppen substituiert sind, ein oder mehrere hydrophobe nichtionische Vinylmonomere und ein oder mehrere assoziative Vinylmonomere umfasst, erhalten werden.

11. Zusammensetzung nach einem der vorhergehenden Ansprüche, umfassend eine oder mehrere von quaternisierter Cellulose abgeleitete kationische Polymere (B), insbesondere ausgewählt aus Hydroxyethylcellulosen der Formel (Ib) und noch besser Polyquaternium-67.

12. Zusammensetzung nach einem der vorhergehenden Ansprüche, umfassend die kationischen Polymere in einer Menge zwischen 0,01 und 2 Gew.-%, insbesondere von 0,025 bis 1 Gew.-%, bevorzugt von 0,05 bis 0,5 Gew.-% und noch besser von 0,06 bis 0,2 Gew.-%, bezogen auf das Gesamtgewicht der Zusammensetzung; und/oder umfassend die assoziativen kationischen Polymere in einer Menge zwischen 0,01 und 2 Gew.-%, insbesondere von 0,025 bis 1 Gew.-%, bevorzugt von 0,05 bis 0,5 Gew.-% und noch besser von 0,06 bis 0,2 Gew.-%, bezogen auf das Gesamtgewicht der Zusammensetzung.

13. Zusammensetzung nach einem der vorhergehenden Ansprüche, umfassend ein oder mehrere nichtionische Tenside, vorzugsweise ausgewählt aus:
Alkoholen, α-Diolen und (C₁-₂₀)Alkylphenolen, wobei diese Verbindungen polyethoxyliert und/oder polypropoxyliert und/oder polyglyceriniert sind, wobei die Zahl der Ethylenoxid- und/oder Propylenoxidgruppen im Bereich von 1 bis 100 liegen kann und die Zahl der Glyceringruppen im Bereich von 2 bis 30 liegen kann; oder wobei diese Verbindungen auch mindestens eine Fettkette mit 8 bis 30 Kohlenstoffatomen und insbesondere 16 bis 30 Kohlenstoffatomen umfassen;
- Kondensaten von Ethylenoxid und Propylenoxid mit Fettalkoholen; polyethoxylierten Fettamiden, vorzugsweise mit 2 bis 30 Ethylenoxideinheiten, polyglycerinierten Fettamiden mit durchschnittlich 1 bis 5 und insbesondere 1,5 bis 4 Glyceringruppen; ethoxylierten Fettsäureestern von Sorbitan, vorzugsweise mit 2 bis 40 Ethylenoxideinheiten, Fettsäureestern von Saccharose, polyoxyalkylenierten und vorzugsweise polyoxyethylenierten Fettsäureestern mit 2 bis 150 mol Ethylenoxid einschließlich oxyethylenierter Pflanzenöle, N-(C₆₋₂₄-Alkyl)glucaminderivaten, Aminoxiden wie (C₁₀₋₁₄-Alkyl)aminoxiden oder N- (C₁₀₋₁₄-Acyl)amino-propylmorpholinoxiden;
- nichtionischen Tensiden vom Alkyl(poly)glykosid-Typ, die insbesondere durch die nachstehend allgemeine Formel (VI) wiedergegeben werden:
R₂₀O-(R₂₁O)ₜ₁-(G₁)ᵥ₁
wobei:
R₂₀ für einen linearen oder verzweigten Alkyl- oder Alkenylrest mit 6 bis 24 Kohlenstoffatomen und insbesondere 8 bis 18 Kohlenstoffatomen oder einen Alkylphenylrest, dessen linearer oder verzweigter Alkylrest 6 bis 24 Kohlenstoffatome und insbesondere 8 bis 18 Kohlenstoffatome umfasst, steht;
R₂₁ für einen Alkylenrest mit 2 bis 4 Kohlenstoffatomen steht,
G₁ für eine Zuckereinheit mit 5 bis 6 Kohlenstoffatomen steht,
t₁ einen Wert im Bereich von 0 bis 10 und vorzugsweise 0 bis 4 bedeutet,
v₁ einen Wert im Bereich von 1 bis 15 und vorzugsweise 1 bis 4 bedeutet;
vorzugsweise wobei:
R₂₀ für einen linearen oder verzweigten, gesättigten oder ungesättigten Alkylrest mit 8 bis 18 Kohlenstoffatomen steht,
R₂₁ für einen Alkylenrest mit 2 bis 4 Kohlenstoffatomen steht,
t₁ einen Wert im Bereich von 0 bis 3, vorzugsweise gleich 0, bedeutet,
G₁ Glucose, Fructose oder Galactose, vorzugsweise Glucose, bedeutet;
der Polymerisationsgrad, d. h. der Wert von v₁, im Bereich von 1 bis 15 und vorzugsweise von 1 bis 4 liegen kann; wobei der mittlere Polymerisationsgrad spezieller zwischen 1 und 2 liegt;
vorzugsweise in einer Menge im Bereich von 0,01 bis 20 Gew.-%, vorzugsweise im Bereich von 0,1 bis 15 Gew.-%, noch besser von 0,2 bis 10 Gew.-% und
bevorzugt von 0,5 bis 5 Gew.-%, bezogen auf das Gesamtgewicht der Zusammensetzung, vorliegend.

14. Zusammensetzung nach einem der vorhergehenden Ansprüche, umfassend Wasser in einem Gehalt, der vorzugsweise im Bereich von 40 bis 95 Gew.-%, insbesondere von 50 bis 90 Gew.-% und noch besser von 60 bis 85 Gew.-%, bezogen auf das Gesamtgewicht der Zusammensetzung, liegt.

15. Zusammensetzung nach einem der vorhergehenden Ansprüche mit einem pH-Wert zwischen 4,5 und 9, insbesondere zwischen 5 und 9, noch besser zwischen 5,5 und 8,5, insbesondere zwischen 6 und 8 und bevorzugt zwischen 6,5 und 7,5 inklusive.

16. Kosmetisches Verfahren zur Behandlung und spezieller zum Waschen und/oder Konditionieren von Keratinmaterialien, insbesondere Keratinfasern, insbesondere dem Haar, bei dem man auf die Keratinmaterialien eine Zusammensetzung nach einem der vorhergehenden Ansprüche aufbringt, gegebenenfalls gefolgt von einer Einwirkungszeit und/oder einem Spülschritt und/oder einem Trocknungsschritt.

## Revendications

1. Composition, comprenant :
- au moins un tensioactif anionique choisi parmi les acides et sels alkyl(amido)éther carboxyliques polyoxyalkylénés en C₆-C₂₄ comprenant de 2 à 50 groupes d'oxydes d'éthylène ;
- au moins un organosilane choisi parmi les composés de formule (I) et/ou des oligomères correspondants :
**R₁Si(OR₂)_{z}(R₃)ₓ(OH)_{y}** **(I)**
dans laquelle :
- R₁ est une chaîne à base d'hydrocarbure saturée, linéaire ou ramifiée en C₁-C₂₂, qui peut être substituée par un groupe amine NH₂ ou NHR (R = alkyle en C₁-C₂₀),
- R₂ représente un groupe alkyle comprenant de 1 à 4 atomes de carbone,
- R₃ représente un groupe alkyle comprenant de 1 à 4 atomes de carbone,
- y est 0,
- z désigne un entier allant de 1 à 3,
- x désigne un entier allant de 0 à 2,
- avec z + x + y = 3 ; et
- au moins une amino silicone comprenant au moins un groupe polyoxyalkyléné, l'amino silicone ou les amino silicones étant présente(s) en une quantité allant de 0,01 % à 5 % en poids, par rapport au poids total de la composition.

2. Composition selon la revendication 1, comprenant au moins un tensioactif anionique choisi parmi les acides alkyl(amido)éther carboxyliques polyoxyalkylénés de formule (1) :
R_{1'}-(OC₂H₄)_{n'}-OCH₂COOA (1)
dans laquelle :
- R_{1'} représente un radical alkyle ou alcényle linéaire ou ramifié en C₆-C₂₄, un radical (C₃-C₉)alkylphényle, un radical R_{2'}CONH-CH₂-CH₂- avec R_{2'} désignant un radical alkyle ou alcényle linéaire ou ramifié en C₉-C₂₁; de préférence R_{1'} est un radical alkyle en C₃-C₂₀, de préférence en C₈-C₁₈, et aryle désigne de préférence phényle,
- n' est un nombre entier ou décimal (valeur moyenne) variant de 2 à 24, de préférence de 2 à 10,
- A désigne H, ammonium, Na, K, Li, Mg ou un radical monoéthanolamine ou triéthanolamine ; préférentiellement, les acides alkyl(amido)éther carboxyliques polyoxyalkylénés de formule (1) dans laquelle :
- R_{1'} désigne un radical alkyle linéaire ou ramifié en C₈-C₂₂, en particulier en C₁₀-C₁₆, voire en C₁₂-C₁₄, ou en variante un radical (C₈-C₉)alkylphényle ;
- A désigne un atome d'hydrogène ou de sodium, et
- n' varie de 2 à 20, de préférence de 2 à 10 ;
plus préférentiellement, les acides alkyl(amido)éther carboxyliques polyoxyalkylénés de formule (1) dans laquelle R_{1'} désigne un radical alkyl en C₁₂, A désigne un atome d'hydrogène ou de sodium et n' varie de 2 à 10.

3. Composition selon l'une quelconque des revendications précédentes, dans laquelle la teneur totale en tensioactifs anioniques va de 1 % à 35 % en poids, en particulier de 3 % à 25 % en poids, mieux encore de 5 % à 22 % en poids, par rapport au poids total de la composition.

4. Composition selon l'une quelconque des revendications précédentes, dans laquelle l'organosilane est choisi parmi les composés de formule (I) et/ou leurs oligomères :
R₁Si(OR₂)_{z}(R₃)ₓ(OH)_{y} (I)
dans laquelle :
- R₁ est une chaîne à base d'hydrocarbure linéaire saturée en C₂-C₁₂, qui peut être substituée par un groupement amine NH₂ ou NHR (R = alkyle en Ci-Ce) ; et
- R₂ représente un groupe alkyle linéaire comprenant de 1 à 4 atomes de carbone, et de préférence le groupe éthyle ou méthyle ; et/ou
- R₃ représente un groupe alkyle linéaire comprenant de 1 à 4 atomes de carbone, et de préférence le groupe éthyle ou méthyle ; et/ou
- z = 3 et donc x = y = 0, ou bien z = 2, x = 1 et y = 0 ;
préférentiellement R₁ représente un groupe amino alcool en C₁-C₆, de préférence en C₂-C₄, z = 3 et x = y = 0.

5. Composition selon l'une quelconque des revendications précédentes, dans laquelle l'organosilane est choisi parmi l'octyltriéthoxysilane (OTES), le dodécyltriéthoxysilane, l'octadécyltriéthoxysilane, l'hexadécyltriéthoxy-silane, le 3-aminopropyl triéthoxysilane (APTES); le méthyltriéthoxysilane (MTES) ; le 2-aminoéthyltriéthoxy-silane (AETES), le 3-aminopropyl méthyldiéthoxysilane, le N-(2-aminoéthyl)-3-aminopropyltriéthoxysilane, le 3-(m-aminophénoxy)propyl-triméthoxysilane, le p-aminophényltriméthoxysilane, le N-(2-aminoéthyl-aminométhyl)phénéthyltriméth-oxysilane, et leurs oligomères ; et plus particulièrement choisi parmi le 3-amino-propyltriéthoxysilane (APTES) et ses oligomères.

6. Composition selon l'une quelconque des revendications précédentes, comprenant l'organosilane ou les organosilanes en une quantité allant de 0,01 % à 10 % en poids, de préférence de 0,1 % à 5 % en poids, encore mieux de 0,2 % à 1 % en poids, par rapport au poids total de la composition.

7. Composition selon l'une quelconque des revendications précédentes, dans laquelle l'amino silicone est choisie parmi les amino silicones polyoxyalkylénées, de type multibloc (AB)n, A étant un bloc polysiloxane et B étant un bloc polyoxyalkylène, et comprenant au moins un groupe amine ; en particulier les silicones constituées de motifs répétitifs de formule (III.1) et/ou de formule (III.2) ci-dessous :
**[-(SiMe₂O)ᵥSiMe₂R₇-N(R₈)-R₉-O(C₃H₆O)ₐ(C₂H₄O)_{b}-R₉-N(R₈)-R₇-]** **(III.1)**
**[-(SiMe₂O)ᵥ-SiMe₂-R₇-N(R₈)-R₉-O(C₃H₆O)ₐ(C₂H₄O)_{b}-]** **(III.2)**
dans lesquelles :
- a est un entier supérieur ou égal à 1, de préférence allant de 5 à 200, voire de 60 à 100, et plus particulièrement allant de 10 à 100 ;
- b est un entier compris entre 0 et 200, de préférence allant de 4 à 100, voire de 5 à 50, plus particulièrement entre 5 et 30 ;
- v est un nombre entier allant de 1 à 10 000, plus particulièrement de 10 à 5 000 ;
- R₈, qui peut être identique ou différent, est un atome d'hydrogène ou un méthyle ; de préférence méthyle ;
- R₇, qui peuvent être identiques ou différents, représentent un radical divalent hydrocarboné en C₂-C₁₂, linéaire ou ramifié, comportant éventuellement un ou plusieurs hétéroatomes tels que l'oxygène ; de préférence, R₇ désigne un radical éthylène, un radical propylène linéaire ou ramifié, un radical butylène linéaire ou ramifié, ou un radical CH₂CH₂CH₂OCH₂CH(OH)CH₂- ; préférentiellement R₇ désigne un radical CH₂CH₂CH₂OCH₂CH (OH) CH₂- ;
- R₉, qui peuvent être identiques ou différents, représentent un radical divalent hydrocarboné en C₂-C₁₂, linéaire ou ramifié, comportant éventuellement un ou plusieurs hétéroatomes tels que l'oxygène ; de préférence, R₉ désigne un radical éthylène, un radical propylène linéaire ou ramifié, un radical butylène linéaire ou ramifié, ou un radical CH₂CH₂CH₂OCH₂CH(OH)CH₂-; préférentiellement R₉ désigne -CH(CH₃)-CH₂- ;
de préférence, les blocs siloxane -(SiMe₂O)ᵥ-représentant 50 % en moles à 95 % en moles du poids total de la silicone, plus particulièrement 70 % en moles à 85 % en moles.

8. Composition selon l'une quelconque des revendications précédentes, comprenant la ou les silicones aminées en une quantité allant de 0,05 % à 2 % en poids, préférentiellement de 0,1 % à 0,5 % en poids, par rapport au poids total de la composition.

9. Composition selon l'une quelconque des revendications précédentes, comprenant un ou plusieurs tensioactifs amphotères, de préférence choisis parmi les alkyl(C₈-C₂₀)bétaïnes, les alkyl(C₈-C₂₀)amidoalkyl(C₁-C₆)bétaïnes, les alkyl(C₈-C₂₀) amphoacétates et les alkyl (C₈-C₂₀) amphodiacétates, et leurs mélanges, et en particulier parmi la cocobétaïne et la cocoamidopropylbétaïne ; de préférence en une quantité allant de 1 % à 15 % en poids, en particulier allant de 2 % à 10 % en poids, préférentiellement de 3 % à 8 % en poids, par rapport au poids total de la composition.

10. Composition selon l'une quelconque des revendications précédentes, comprenant un ou plusieurs polymères cationiques; de préférence choisis parmi les polymères associatifs cationiques ; et encore mieux choisis parmi :
- (A) les polyuréthanes associatifs cationiques, de formule générale (Ia) ci-dessous :
R-X-(P)n-[L-(Y)m]r-L'-(P')p-X'-R'
dans laquelle :
R et R', qui peuvent être identiques ou différents, représentent un groupement hydrophobe ou un atome d'hydrogène ;
X et X', qui peuvent être identiques ou différents, représentent un groupement comportant une fonction amine portant ou non un groupement hydrophobe, ou encore le groupement L" ;
L, L' et L", qui peuvent être identiques ou différents, représentent un groupement dérivé d'un diisocyanate ;
P et P', qui peuvent être identiques ou différents, représentent un groupement comportant une fonction amine portant ou non un groupement hydrophobe ;
Y représente un groupement hydrophile ;
r est un entier compris inclusivement entre 1 et 100, de préférence entre inclusivement 1 et 50 et en particulier entre inclusivement 1 et 25,
n, m, et p valent chacun indépendamment des autres entre inclusivement 0 et 1 000 ;
la molécule contenant au moins une fonction amine protonée ou quaternisée et au moins un groupement hydrophobe ;
- (B) les dérivés de cellulose quaternisée, et en particulier :
- i) les celluloses quaternisées modifiées par des groupements comportant au moins une chaîne grasse, tels que les groupes alkyle linéaire ou ramifié, arylalkyle linéaire ou ramifié ou alkylaryle linéaire ou ramifié comportant au moins 8 atomes de carbone, ou des mélanges de ceux-ci ;
- ii) les hydroxyéthylcelluloses quaternisées modifiées par des groupements comportant au moins une chaîne grasse, tels que les groupes alkyle linéaire ou ramifié, arylalkyle linéaire ou ramifié ou alkylaryle linéaire ou ramifié comportant au moins 8 atomes de carbone, ou des mélanges de ceux-ci ;
- iii) les hydroxyéthylcelluloses de formule (Ib) : 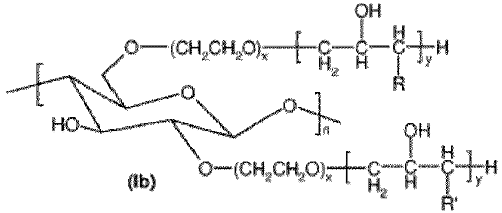 dans laquelle :
R et R', qui peuvent être identiques ou différents, représentent un groupement ammonium RaRbRcN⁺-, Q⁻ dans lequel Ra, Rb, Rc, qui peuvent être identiques ou différents représentent un atome d'hydrogène, un groupement alkyle, linéaire ou ramifié, en C₁-C₃₀ et préférentiellement C₁-C₂₀, tel que méthyle ou dodécyle ; et Q⁻ représente un contre-ion anionique tel qu'un halogénure, par exemple un chlorure ou bromure ; et n, x et y, qui peuvent être identiques ou différents, représentent un nombre entier compris entre 1 et 10 000, les radicaux alkyles portés par les celluloses i) ou hydroxyéthylcelluloses quaternisées ii) ci-dessus comportent de préférence de 8 à 30 atomes de carbone, les radicaux aryle désignent de préférence les groupements phényle, benzyle, naphtyle ou anthryle ;
- (C) les polyvinyllactames cationiques, en particulier ceux comprenant des motifs issus de :
- a) au moins un monomère de type vinyllactame ou alkylvinyllactame ;
- b) au moins un monomère de structure (Ic) ou (IIc) ci-dessous : 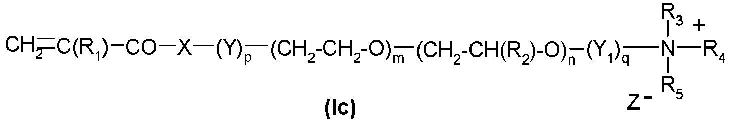 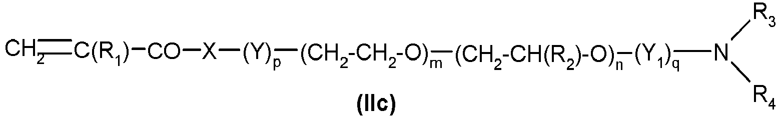 dans lesquelles :
- X désigne un atome d'oxygène ou un radical NR₆,
- R₁ et R₆ désignent, indépendamment l'un de l'autre, un atome d'hydrogène ou un radical alkyl linéaire ou ramifié en C₁-C₅,
- R₂ désigne un radical alkyle linéaire ou ramifié en C₁-C₄,
- R₃, R₄ et R₅ désignent, indépendamment l'un de l'autre, un atome d'hydrogène, un radical alkyle linéaire ou ramifié en C₁-C₃₀ ou un radical de formule (IIIc) :
**-(Y₂)ᵣ-(CH₂-CH(R₇)-O)ₓ-R₈** **(IIIc)**
- Y, Y₁ et Y₂ désignent, indépendamment l'un de l'autre, un radical alkylène linéaire ou ramifié en C₂-C₁₆,
- R₇ désigne un atome d'hydrogène, ou un radical alkyle linéaire ou ramifié en C₁-C₄ ou un radical hydroxyalkyle linéaire ou ramifié en C₁-C₄,
- R₈ désigne un atome d'hydrogène ou un radical alkyle linéaire ou ramifié en C₁-C₃₀,
- p, q et r désignent, indépendamment l'un de l'autre, soit la valeur zéro, soit la valeur 1,
- m et n désignent, indépendamment l'un de l'autre, un nombre entier allant inclusivement de 0 à 100,
- x désigne un nombre entier allant inclusivement de 1 à 100,
- Z désigne un contre-ion anionique d'acide organique ou minéral, tel qu'un halogénure, par exemple un chlorure ou un bromure ou un mésylate ;
sous réserve que :
- l'un au moins des substituants R₃, R₄, R₅ ou R₈ désigne un radical alkyle linéaire ou ramifié en C₉-C₃₀,
- si m ou n est différent de zéro, alors q est égal à 1,
- si m ou n sont égaux à zéro, alors p ou q est égal à 0,
- (D) les polymères cationiques obtenus par polymérisation d'un mélange de monomères comprenant un ou plusieurs monomères vinyliques substitués par un ou plusieurs groupes amino, un ou plusieurs monomères vinyliques non ioniques hydrophobes, et un ou plusieurs monomères vinyliques associatifs.

11. Composition selon l'une quelconque des revendications précédentes, comprenant un ou plusieurs polymères cationiques (B) issus de cellulose quaternisée, particulièrement choisis parmi les hydroxyéthylcelluloses de formule (Ib) et encore mieux le polyquaternium-67.

12. Composition selon l'une quelconque des revendications précédentes, comprenant les polymères cationiques en une quantité comprise entre 0,01 % et 2 % en poids, en particulier de 0,025 % à 1 % en poids, préférentiellement de 0,05 % à 0,5 % en poids, encore mieux de 0,06 % à 0,2
% en poids, par rapport au poids total de la composition ; et/ou comprenant les polymères cationiques associatifs en une quantité comprise entre 0,01 % et 2 % en poids, en particulier de 0,025 % à 1 % en poids, préférentiellement de 0,05 % à 0,5 % en poids, encore mieux de 0,06 % à 0,2 % en poids, par rapport au poids total de la composition.

13. Composition selon l'une quelconque des revendications précédentes, comprenant un ou plusieurs tensioactifs non ioniques, de préférence choisis parmi :
- les alcools, les α-diols et les alkyl(C₁-C₂₀)phénols, ces composés étant polyéthoxylés et/ou polypropoxylés et/ou polyglycérolés, le nombre de groupements oxyde d'éthylène et/ou oxyde de propylène pouvant aller de 1 à 100, et le nombre de groupements glycérol pouvant aller de 2 à 30 ; ou bien ces composés comprenant au moins une chaîne grasse comportant de 8 à 30 atomes de carbone, en particulier de 16 à 30 atomes de carbone ;
- les condensats d'oxyde d'éthylène et d'oxyde de propylène avec des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4 ; les esters d'acides gras du sorbitane éthoxylés ayant de préférence de 2 à 40 motifs d'oxyde d'éthylène, les esters d'acides gras du saccharose, les esters d'acides gras polyoxyalkylénés, de préférence polyoxyéthylénés ayant de 2 à 150 moles d'oxyde d'éthylène, y compris les huiles végétales oxyéthylénées, les dérivés de N-(alkyl en C₆-₂₄)glucamine, les oxydes d'amines tels que les oxydes d' (alkyl en C₁₀-₁₄) amines ou les oxydes de N-(acyl en C₁₀-₁₄)-aminopropylmorpholine ;
- les tensioactifs non ioniques de type alkyl(poly)glycoside, en particulier représentés par la formule générale (VI) ci-dessous :
R₂₀O-(R₂₁O)ₜ₁-(G₁)ᵥ₁
dans laquelle :
R₂₀ représente un radical alkyle ou alcényle linéaire ou ramifié comportant 6 à 24 atomes de carbone, en particulier 8 à 18 atomes de carbone, ou un radical alkylphényle dont le radical alkyle linéaire ou ramifié comporte 6 à 24 atomes de carbone, en particulier 8 à 18 atomes de carbone ;
R₂₁ représente un radical alkylène comportant 2 à 4 atomes de carbone,
G₁ représente un motif sucre comportant 5 à 6 atomes de carbone,
t₁ désigne une valeur allant de 0 à 10, de préférence de 0 à 4,
v₁ désigne une valeur allant de 1 à 15, de préférence de 1 à 4 ;
de préférence, dans laquelle :
R₂₀ désigne un radical alkyle saturé ou insaturé, linéaire ou ramifié comportant de 8 à 18 atomes de carbone,
R₂₁ représente un radical alkylène comportant 2 à 4 atomes de carbone,
t₁ désigne une valeur allant de 0 à 3, de préférence égale à 0,
G₁ désigne le glucose, le fructose ou le galactose, de préférence le glucose ;
le degré de polymérisation, c'est-à-dire la valeur de v₁, pouvant aller de 1 à 15, de préférence de 1 à 4 ; le degré moyen de polymérisation étant plus particulièrement compris entre 1 et 2 ;
de préférence présents en une quantité allant de 0,01 % à 20 % en poids, en particulier allant de 0,1 % à 15 % en poids, préférentiellement de 0,2 % à 10 %, et en particulier entre 0,5 % et 5 % en poids, par rapport au poids total de la composition.

14. Composition selon l'une quelconque des revendications précédentes, comprenant de l'eau en une teneur allant de préférence de 40 % à 95 % en poids, en particulier de 50 % à 90 % en poids, mieux de 60 % à 85 % en poids, par rapport au poids total de la composition.

15. Composition selon l'une quelconque des revendications précédentes, présentant un pH compris inclusivement entre 4,5 et 9, en particulier entre 5 et 9, encore mieux entre 5,5 et 8,5, en particulier entre 6 et 8, et préférentiellement entre 6,5 et 7,5.

16. Procédé cosmétique pour le traitement, et plus particulièrement pour le lavage et/ou le conditionnement, de matières kératiniques, en particulier de fibres kératiniques, en particulier des cheveux, qui comprend l'application sur lesdites matières kératiniques d'une composition selon l'une quelconque des revendications précédentes, suivie éventuellement par un temps de pose et/ou une étape de rinçage et/ou une étape de séchage.
